# EUROPÄISCHE PATENTSCHRIFT

(11) **EP 3 892 165 B1**
(45) Veröffentlichungstag und Bekanntmachung des Hinweises auf die Patenterteilung: **02.11.2022**
(21) Anmeldenummer: 21166975.9
(22) Anmeldetag: 06.04.2021
(51) Int. Cl.: A47G 25/90, A61F 13/08

(54) **ANZIEHHILFE FÜR FUSSSTRÜMPFE UND VERFAHREN ZUR UNTERSTÜTZUNG BEIM ANZIEHEN EINES FUSSSTRUMPFES**
TIGHTENING AID FOR STOCKINGS AND METHOD OF ASSISTING WITH THE WEARING OF A STOCKING
AIDE À L'ENFILAGE DES CHAUSSETTES ET PROCÉDÉ D'AIDE À L'ENFILAGE D'UNE CHAUSSETTE

(30) Priorität: 12.04.2020 DE 102020204660; 01.05.2020 DE 102020205571
(43) Veröffentlichungstag der Anmeldung: 13.10.2021
(73) Patentinhaber: FXF GmbH, 92224 Amberg (DE)
(72) Erfinder: FISCHER, Franz, 92224 Amberg (DE)
(74) Vertreter: Benninger, Johannes

(56) Entgegenhaltungen:
- EP-B1- 3 280 372
- DE-A1-102004 001 905
- US-A- 4 260 083
- US-A1- 2016 302 602

## Beschreibung

Die vorliegende Erfindung betrifft einen Strumpfanzieher, d.h. eine Anziehhilfe zur Ermöglichung oder Erleichterung des Überstreifens eines Strumpfes über einen menschlichen Fuß, wobei der Strumpf insbesondere ein Kompressionsstrumpf oder ein Kompressions-Zehenstrumpf sein kann. Zudem betrifft die Erfindung ein Verfahren zum erleichterten Anziehen oder zur Unterstützung beim Anziehen eines solchen Kompressionsstrumpfes oder Kompressions-Zehenstrumpfes.

Socken und Strümpfe können unterschiedlichen Zwecken dienen, werden aber in aller Regel als Schutz gegen Kälte sowie als Zwischenschicht beim Tragen von festem Schuhwerk verwendet. Bei solchen herkömmlichen Socken und Strümpfe wird aus Komfortgründen in aller Regel ein straffer und faltenfreier, aber nicht zu fester Sitz bevorzugt, was bei den meist relativ dünnen und ausreichend elastischen Socken und Strümpfen normalerweise problemlos zu gewährleisten ist.

Etwas anders geartet sind die Zusammenhänge beim Tragen von solchen Strümpfen, die vorrangig oder ausschließlich therapeutischen Zwecken dienen sollen, und die deshalb nicht nur eine definierte Materialelastizität mitbringen, sondern die auch besonders straff sitzen sollen, da ihre Materialelastizität auf den mit dem Strumpf ausgestatteten Fuß, das Fußgelenk und/oder Unterschenkel übertragen werden soll. Die dem Strumpf innewohnende Materialelastizität soll dabei als komprimierende Kraftwirkung auf die damit bedeckten Bereiche des Fußes, des Fußgelenks und/oder des Unterschenkels einwirken.

Solche Stütz- oder Kompressionsstrümpfe werden nicht nur von Menschen mit erhöhter Thrombosegefahr, sondern typischerweise auch von Schlaganfallpatienten mit einseitiger Beeinträchtigung ihrer Motorik, herkömmlicherweise auch als halbseitige Lähmung oder Teillähmung bezeichnet, getragen. Da bei solchen Patienten nicht nur die Motorik aufgrund gestörter Nervensignalübertragung und/oder Nervensignalverarbeitung beeinträchtigt ist, sondern auch manche vegetativen Körperfunktionen wie etwa der Lymphabfluss aus den betroffenen Gliedmaßen, hat es sich als sinnvoll erwiesen, Letzteres durch das Tragen von Stützstrümpfen oder Kompressionsstrümpfen zumindest teilweise therapierend auszugleichen.

Zum Zwecke des erleichterten Anziehens von Handschuhen offenbart die US 2016/0302602 A1 eine schablonenartige Anziehhilfe aus Blech oder einem anderen Flachmaterial, über die ein Handschuh gezogen wird, der von Fingerabschnitten der Schablone ausgefüllt und stabilisiert ist. Die Schablone wird mittels eines Durchbruchs an geeigneter Stelle fixiert oder mit der jeweils anderen Hand, der kein Handschuh übergestreift werden soll oder die bereits mit einem Handschuh bedeckt ist, gehalten.

Eine ähnliche Anziehhilfe findet sich durch US D 761 517 S offenbart.

Ähnliche, aber angepasste Anziehhilfen zur Verwendung beim erleichterten Anziehen von Kompressionsstrümpfen, wären denkbar. Bekannt geworden sind solche Strumpf-Anziehhilfen bislang jedoch nicht.

Trotz aller aus dem Stand der Technik bekannten Anziehhilfen besteht weiterhin ein Bedarf an mechanischer Unterstützung beim Anziehen von elastischen Strümpfen oder Kompressionsstrümpfen, die sich ohne zusätzliche Unterstützung nur schwer anziehen lassen, insbesondere wenn der Träger über eine eingeschränkte Beweglichkeit oder Motorik verfügt.

Aus diesem Grund kann das vorrangige Ziel der vorliegenden Erfindung darin gesehen werden, eine Anziehhilfe zur Verfügung zu stellen, die es einem Träger ermöglicht, auch solche Socken oder Strümpfe anzulegen, die zwar dehnbar sind, die jedoch über besonders hohe elastische Rückstellkräfte verfügen. Dies betrifft insbesondere Kompressionsstrümpfe, die zu therapeutischen Zwecken getragen werden sollen, die sich jedoch oftmals nur unter größeren Schwierigkeiten anziehen und über den Fuß streifen lassen, insbesondere vom Träger selbst, d.h. ohne Unterstützung durch eine dritte Person.

Ein weiteres Ziel der Erfindung besteht darin, ein verbessertes Verfahren zum erleichterten Anziehen eines Kompressionsstrumpfes und/oder zur Unterstützung beim Überstreifen eines Kompressionsstrumpfes über einen menschlichen Fuß unter Verwendung einer solchen Anziehhilfe zur Verfügung zu stellen.

Die oben definierten Ziele werden durch die Gegenstände der unabhängigen Ansprüche erreicht. Weitere vorteilhafte Ausgestaltungen werden durch die Unteransprüche beschrieben.

Zur Erreichung zumindest eines der oben genannten Ziele schlägt die vorliegende Erfindung eine Anziehhilfe zur Ermöglichung oder Erleichterung des Überstreifens eines Strumpfs über einen menschlichen Fuß mit den Merkmalen des unabhängigen Anspruchs 1 vor. Bei dem überzustreifenden oder anzuziehenden Strumpf kann es sich insbesondere um einen Kompressionsstrumpf oder Kompressions-Zehenstrumpf handeln, der im entspannten Zustand ohne darin aufgenommenen Fuß zumindest abschnittsweise ein kleineres Volumen bereithält oder einnimmt als im gedehnten, über den Fuß gezogenen Zustand, da hier die Vorteile der erfindungsgemäßen Anziehhilfe besonders deutlich zum Tragen kommen und augenscheinlich werden.

Die erfindungsgemäße Anziehhilfe umfasst eine aus widerstandsfähigem und zumindest weitgehend formstabilem Flachmaterial gebildete und gefertigte Schablone mit Umrisskonturen eines ausgestreckten und/oder flach auf einer Unterlage liegenden oder stehenden menschlichen Fußes mit zumindest leicht voneinander abgespreizten und/oder seitlich voneinander beabstandeten Zehenabschnitten.

Wenn hier und in der weiteren Folge teilweise die vertrauten Begrifflichkeiten des menschlichen Fußes verwendet und auf die verschiedenen Abschnitte der erfindungsgemäßen Schablone bezogen werden, so soll dies in erster Linie die Anschaulichkeit verbessern und nicht zu Verwechslungen führen. Es ist im Einzelfall aus dem Zusammenhang zu erschließen, ob bei den gleichen oder sehr ähnlich klingenden Begrifflichkeiten von der Schablone oder von einem realen Fuß die Rede ist.

So meint der im Zusammenhang mit der Schablone verwendete Begriff der Fuß- oder Sohlenfläche deren mittleren Bereich, auf dem der menschliche Fuß zum Liegen oder Stehen käme, wenn er korrespondierend zu den Umrisskonturen auf der Schablone abgelegt oder abgesetzt würde. Die Zehenabschnitte der Schablone meinen die schmaleren Bereiche, auf denen die Zehen des menschlichen Fußes zum Liegen kämen, wenn sie in leicht voneinander gespreizter Fuß- und Zehenstellung mit auf der mittleren Fläche der Schablone liegender oder stehender Fuß- oder Sohlenfläche entsprechend den Umrisskonturen der Schablone abgelegt würden.

Weiterhin umfasst die Schablone der erfindungsgemäßen Anziehhilfe zumindest an einer Längsseite der Sohlenfläche der Schablone einen zumindest abschnittsweise hochgezogenen Rand. Dieser Rand kann wahlweise auch zu beiden Längsseiten der Sohlenfläche der Schablone vorgesehen sein. Allerdings sei an dieser Stelle betont, dass die Anziehhilfe bereits auch mit einseitig vorhandenem Rand voll funktionsfähig ist. Zur Kontur und Formgebung des hochgezogenen Randes oder der hochgezogenen Ränder sei auf die nachfolgenden Beschreibungsabschnitte verwiesen.

Mit diesem zumindest einseitig an einer Längsseite der Schablone vorhandenen hochgezogenen Rand geht die erfindungsgemäße Anziehhilfe deutlich über die bisher bekannten Anziehhilfen für Handschuhe hinaus, wie sie etwa in der US 2016/0302602 A1 offenbart sind. Während diese bekannte Anziehhilfe durchaus ihren Zweck erfüllen kann, wenn es sich bei dem Handschuh um einen solchen aus Leder oder Kunstleder oder einem anderen nichtelastischen Material handelt, kann sie als Anziehhilfe für elastische Strumpfvarianten, deren Volumen im entspannten Zustand ohne darin aufgenommenen Fuß normalerweise deutlich gegenüber dem gedehnten Zustand, in dem sie getragen werden, reduziert ist, kaum noch oder nur sehr unzureichend das Anziehen und Überstreifen des Strumpfes erleichtern.

Da die bekannten Anziehhilfen für Handschuhe - etwa gemäß US 2016/0302602 A1 oder gemäß US D 761 517 S - keine Öffnung zum Einführen der Finger bereithalten, hat eine diese Anziehhilfen benutzende Person große Mühen, gegen den Widerstand eines über die Anziehhilfe gezogenen und dabei nur teilweise gedehnten Kompressionshandschuhs diesen weiter aufzudehnen und die Finger bis zum endgültigen Sitz in den Fingerschläuchen des Handschuhs hineinzuschieben. Würde eine solche Anziehhilfe in modifizierter Version für Strümpfe verwendet, so hätte Entsprechendes für das Einschieben der Zehen zu gelten, die keine brauchbare Öffnung vorfänden, in die sie hineingleiten könnten.

Hinzu kommt, dass die hier interessierenden Kompressionsstrümpfe oftmals von Patienten mit erheblichen motorischen Einschränkungen getragen werden, die gegenüber gesunden Personen im deutlichen Nachteil sind, was ihre Beweglichkeit und ihre Feinmotorik im Fuß und in den Zehen betrifft.

Außerdem umfasst die Schablone der erfindungsgemäßen Anziehhilfe wenigstens ein Halte-, Fixierungs- und/oder Verankerungselement zur zumindest temporären Fixierung oder Abstützung der Schablone beim Einführen des mit dem Strumpf zu bedeckenden Fußes gegen elastische Rückstellkräfte des über die Schablone gezogenen und dabei entsprechend den Umrisskonturen der Schablone gedehnten Strumpfes oder Kompressionsstrumpfes bzw. des Kompressions-Zehenstrumpfes.

Zur Erreichung zumindest eines oder mehrerer der oben genannten Ziele schlägt die Erfindung außerdem ein Verfahren zum erleichterten Anziehen eines Kompressionsstrumpfes oder Kompressions-Zehenstrumpfes und/oder zur Unterstützung beim Überstreifen eines Kompressionstrumpfes oder Kompressions-Zehenstrumpfes über einen menschlichen Fuß mit den Merkmalen des unabhängigen Verfahrensanspruchs vor.

Bei diesem erfindungsgemäßen Verfahren kann insbesondere eine mechanische Anziehhilfe verwendet werden, wie sie zuvor definiert und beschrieben wurde. Bei dem Verfahren kann wahlweise auch eine mechanische Anziehhilfe gemäß einer der nachfolgend erläuterten Ausführungsvarianten oder Abwandlungen zum Einsatz kommen.

Unabhängig von der jeweils eingesetzten Anziehhilfe sieht das Verfahren zumindest die nachfolgend erläuterten Verfahrensschritte vor. So beginnt das Verfahren fast zwangsläufig mit dem Überstreifen des Kompressionsstrumpfes oder Kompressions-Zehenstrumpfes über eine Schablone, welche Umrisskonturen eines ausgestreckten und/oder flach auf einer Unterlage liegenden oder stehenden menschlichen Fußes mit zumindest leicht voneinander abgespreizten und/oder seitlich voneinander beabstandeten Zehenabschnitten aufweist, wobei die Zehenabschnitte der Schablone in die Zehenschläuche des Kompressionsstrumpfes oder Kompressions-Zehenstrumpfes eintauchen. Erst wenn dieser Bereitstellungszustand hergestellt ist, kann die Person, die sich den Kompressionsstrumpf oder den Kompressions-Zehenstrumpf anziehen und überstreifen will, mit dem eigentlichen Anziehvorgang beginnen.

Der beschriebene Bereitstellungszustand, bei dem der Kompressionsstrumpf oder den Kompressions-Zehenstrumpf über die Schablone gezogen und von dieser aufgespannt ist, kann von jeder beliebigen dritten Person hergestellt werden, so bspw. von einer Pflegeperson, die den Bereitstellungszustand für einen Patienten mit eingeschränkter Motorik herstellt. Allerdings ist die Schablone so gut und vergleichsweise einfach handhabbar, dass auch ein Patient mit zumindest teilweise eingeschränkter Handmotorik und/oder eingeschränkter Fußmotorik den Bereitstellungszustand ohne unzumutbare Mühen herstellen kann.

Zum Herstellen des beschriebenen Bereitstellungszustandes kann außerdem das nachfolgende Positionieren und Arretieren und/oder Fixieren der in den Kompressionsstrumpf oder den Kompressions-Zehenstrumpf eingetauchten Schablone an einem Gegenlager hinzugerechnet werden, da dieses Arretieren für das nachfolgende Hineinschieben des mit dem Strumpf zu bedeckenden Fußes in den von der Schablone aufgespannten Kompressionsstrumpf oder den Kompressions-Zehenstrumpf unbedingt sinnvoll ist, um ein Ausweichen der Schablone zumindest in einer Einschubrichtung, d.h. in einer von der Ferse zu den Zehen weisenden Richtung effektiv verhindern zu können.

Nach einer passenden Positionierung und/oder Arretierung der Schablone an einem Gegenlager, wie es bspw. durch eine Tischkante, durch eine Treppenstufe, einen entsprechend hierfür vorgesehenen Haltezapfen o. dgl. gebildet sein kann, kann das Einschieben des mit dem Kompressionsstrumpf oder den Kompressions-Zehenstrumpf auszustattenden menschlichen Fußes in den durch die Schablone in Form gebrachten und durch wenigstens einen an einer Seite hochgezogenen Rand oder durch die randseitig hochgezogenen Ränder der Schablone in einer Richtung entgegen der Einschubrichtung geöffneten Kompressionsstrumpf oder den Kompressions-Zehenstrumpf oder im vorderen, den Fuß bedeckenden Bereich des Kompressionsstrumpf oder den Kompressions-Zehenstrumpf erfolgen. Während dieses Vorgangs des Einschiebens des Fußes wird der Kompressionsstrumpf oder den Kompressions-Zehenstrumpf durch die keilförmige Gestaltung des Randes oder der randseitig hochgezogenen Ränder der Schablone dort gehalten.

Sobald der Fuß mit seinen Zehen vollständig im Kompressionsstrumpf oder den Kompressions-Zehenstrumpf eingetaucht ist, wobei die Zehen vorzugsweise an die geschlossenen Enden der Zehenschläuche des Strumpfes stoßen oder wahlweise durch offene Enden der Zehenschläuche hindurchtreten, erfolgt schließlich das Abziehen des vollständig oder weitgehend durch die Zehen und den Fuß ausgefüllten Kompressionsstrumpf oder den Kompressions-Zehenstrumpf von der Schablone durch oder unter gleichzeitigem Lösen der am Rand oder an den hochgezogenen Rändern der Schablone gehaltenen Bereiche des Kompressionsstrumpfes oder des Kompressions-Zehenstrumpfes.

Es sei an dieser Stelle ausdrücklich erwähnt, dass alle Aspekte und Ausführungsvarianten, die im Zusammenhang mit der erfindungsgemäßen Anziehhilfe erläutert wurden und nachfolgend erläutert werden, gleichermaßen Teilaspekte des erfindungsgemäßen Verfahrens zum erleichterten Anziehen eines Kompressionsstrumpfes oder Kompressions-Zehenstrumpfes und/oder zur Unterstützung beim Überstreifen eines Kompressionsstrumpfes oder Kompressions-Zehenstrumpfes über einen menschlichen Fuß betreffen oder bilden können. Wenn daher an einer Stelle bei der Beschreibung oder auch bei den Anspruchsdefinitionen zur erfindungsgemäßen Anziehhilfe von bestimmten Aspekten und/oder Zusammenhängen und/oder Wirkungen die Rede ist, so gilt dies gleichermaßen für das erfindungsgemäße Verfahren.

In umgekehrter Weise gilt dasselbe, so dass auch alle Aspekte und Ausführungsvarianten, die im Zusammenhang mit dem erfindungsgemäßen Verfahren erläutert wurden oder nachfolgend erläutert werden, gleichermaßen Teilaspekte der erfindungsgemäßen Anziehhilfe betreffen oder sein können. Wenn daher an einer Stelle bei der Beschreibung oder auch bei den Anspruchsdefinitionen zum erfindungsgemäßen Verfahren von bestimmten Aspekten und/oder Zusammenhängen und/oder Wirkungen die Rede ist, so gilt dies gleichermaßen für die erfindungsgemäße Anziehhilfe.

Ein hier ganz besonders zu betonender Vorteil der erfindungsgemäßen Anziehhilfe sowie des Verfahrens zur Unterstützung des Überstreifens eines Kompressionsstrumpfes oder Kompressions-Zehenstrumpfes besteht darin, dass mit einer solchen Anziehhilfe und durch korrekte Anwendung des erfindungsgemäßen Verfahrens gewährleistet werden kann, dass der Kompressionsstrumpf vollständig angezogen werden kann und solchermaßen seine therapeutisch wichtigen Wirkungen in optimaler Weise entfalten kann.

Da viele motorische Einschränkungen bei zahlreichen Patienten nur einen Teil ihrer Beschwerden bilden und oftmals nur Symptome anderer Einschränkungen sind, ist es insbesondere beim medizinisch indizierten Tragen von Kompressionsstrümpfen und Kompressions-Zehenstrümpfen wichtig, dass die komprimierende Wirkung die gesamten Extremitäten bis hin zu allen Gliedmaßen, d.h. bis zu den Zehenspitzen gleichermaßen erreicht. Bei einem nicht korrekt übergestreiften oder nicht vollständig angezogenen Kompressionsstrumpf oder Kompressions-Zehenstrumpf bestünde bspw. die Gefahr, dass manche Fußbereiche oder einzelne Zehen nicht in gleichem Ausmaß vom Strumpf komprimiert sind wie die übrigen Bereiche. In diesen Fuß- oder Zehenbereichen könnten dann aufgrund der komprimierten übrigen Bereiche Stauerscheinungen drohen, da etwa der korrekte Lymphabfluss gestört sein könnte.

Die beschriebenen Ungenauigkeiten und Nachlässigkeiten beim Überstreifen und auch beim Tragen von Kompressionsstrümpfen und von Kompressions-Zehenstrümpfen gilt es unbedingt zu vermeiden, da ansonsten schwerwiegendere Gesundheitsgefahren drohen können. Diese potentiellen Gesundheitsgefahren können in vorteilhafter Weise durch korrekte Verwendung der erfindungsgemäßen Anziehhilfe und durch korrekte Anwendung des erfindungsgemäßen Verfahrens zumindest deutlich reduziert und im Idealfall nahezu vollständig vermieden werden.

Bei einer besonders sinnvollen Variante der erfindungsgemäßen Anziehhilfe kann der zumindest an einer Längsseite der Fuß- oder Sohlenfläche der Schablone hochgezogene Rand in Richtung zum Fersenbereich höher gezogen sein als in einer Richtung zu den Zehenabschnitten der Schablone. Auf diese Weise wird eine Öffnung des Kompressionsstrumpfes, die im Bereich des Fersenbereiches und des Fußgelenks liegen kann, weiter geöffnet als die Bereiche des Kompressionsstrumpfes, die näher an den Zehenabschnitten liegen, so dass der Fuß in die Strumpföffnung leichter hineingeführt werden kann.

Insbesondere kann vorgesehen sein, dass der zumindest an einer Längsseite der Sohlenfläche der Schablone hochgezogene Rand eine keil- oder dreieckförmige Kontur aufweist, deren kurze Seite im Bereich des Fersenbereiches oder des Fußgelenks rechtwinkelig zur Sohlenfläche steht oder mit einer zur Sohlenfläche senkrechten Achse einen spitzen Winkel einschließt.

Ebenso denkbar ist es, dass der zumindest an einer Längsseite der Sohlenfläche der Schablone hochgezogene Rand eine keil- oder dreieckförmige Kontur aufweist, deren lange Seite an die im Bereich des Fersenbereiches oder des Fußgelenks befindliche kurze Seite anschließt und in spitzem Winkel in Richtung zu einem randseitigen Zehenabschnitt hin ausläuft.

Es kann außerdem vorgesehen sein, dass der zum randseitig befindlichen Zehenabschnitt hin auslaufende hochgezogene Rand in den Zehenabschnitt hineinreicht oder dort ausläuft.

Vorzugsweise kann bei der Schablone der Anziehhilfe vorgesehen sein, dass die freien Enden der Zehenabschnitte jeweils einen Radius aufweisen, insbesondere einen Halbkreisradius mit einem Durchmesser, der in etwa der Breite des jeweiligen Zehenabschnittes entspricht. Eine solche Konturierung ist deshalb sinnvoll, weil auch die geschlossenen Enden der Zehenschläuche üblicher Kompressionsstrümpfe bzw. üblicher Kompressions-Zehenstrümpfe eine gewölbte Kontur aufweisen, so dass eine mit Rundung versehene Konturierung der Schablone der Formgebung der Strümpfe entspricht.

Eine besonders vorteilhafte Ausführungsvariante der erfindungsgemäßen Anziehhilfe sieht vor, dass an beiden gegenüberliegenden und voneinander beabstandeten Längsseiten der Sohlenfläche der Schablone jeweils zumindest abschnittsweise hochgezogene Ränder aufweist.

Diese hochgezogenen Ränder können in etwa parallel zueinander stehen und ähnliche oder in etwa deckungsgleiche Umrisskonturen aufweisen. Normalerweise streben die hochgezogenen Ränder jedoch, der Kontur des von der Ferse zu den Zehen hin breiter werdenden menschlichen Fußes folgend, in Richtung zu den Zehenabschnitten V-förmig auseinander.

Sinnvollerweise sind die hochgezogenen Ränder in etwa senkrecht zur Sohlenfläche der Schablone ausgerichtet. Sie können jedoch auch jeweils nach außen geneigt sein, so dass eine wannenartige Kontur gebildet ist, was ggf. das Einführen des Fußes bei entsprechend weiter geöffnetem Kompressionsstrumpf erleichtern kann.

Wenn oben von keilförmigen oder dreieckförmigen hochgezogenen Rändern oder Randabschnitten der Schablone die Rede ist, so schließt dies keinesfalls Varianten mit leicht abweichenden Konturen aus, so dass die obere Längskante der hochgezogenen Ränder auch bspw. eine wellige Kontur, eine leicht konvexe oder auch eine leicht konkave Wölbung aufweisen kann.

Bei der erfindungsgemäßen Anziehhilfe kann weiterhin vorgesehen sein, dass der an den Zehenabschnitt der Schablone für den kleinen Zeh des Fußes grenzende hochgezogene Rand ungefähr am Übergang des entsprechenden Zehenabschnittes zu dem an dessen freien Ende befindlichen Radius oder Halbkreisradius endet oder dort ausläuft. In gleicher Weise kann der an den Zehenabschnitt der Schablone für den Großzeh des Fußes grenzende hochgezogene Rand ungefähr am Übergang des entsprechenden Zehenabschnittes zu dem an dessen freien Ende befindlichen Radius oder Halbkreisradius enden oder dort auslaufen.

Bei einer besonders vorteilhaften Variante der erfindungsgemäßen Anziehhilfe kann der wenigstens eine hochgezogene Rand oder zumindest einer der beiden hochgezogenen Ränder an der oberen Kante der im Bereich der Ferse oder des Fußgelenks befindlichen kurzen Seite abgestuft ausgebildet sein, wodurch der Eckbereich im Übergang zur langen keilförmigen Seite des Randes eine stufenartige Aussparung bildet. Eine solche Abstufung in der Höhe des wenigstens einen Randes oder beider Ränder kann helfen, dass der Kompressionsstrumpf zunächst an seinem Platz gehalten wird und nicht nach vorne in Richtung der Zehen abrutscht, wenn zunächst erst die Zehen in die Strumpföffnung eingeschoben werden.

Der nach vorne in Zehenrichtung tiefer werdende Rand oder die nach vorne in Zehenrichtung tiefer werdenden Ränder folgen der dreidimensionalen Kontur eines jeden Strumpfes, der in Richtung zu den Zehen flacher und in seinem Volumen etwas kleiner wird.

Sobald die Zehen des in den Kompressionsstrumpf oder Kompressions-Zehenstrumpf eingeschobenen menschlichen Fußes die Zehenschläuche des Strumpfes erreichen, wölbt der ein größeres Volumen beanspruchende Fußgelenkbereich mit dem oftmals voluminöseren Fersenbereich die Einschlüpföffnung des Strumpfes stärker nach oben, d.h. in einer Richtung senkrecht zur Oberfläche der Schablone, die sich zwischen den seitlichen hochgezogenen Rändern befindet, bis sich schließlich beim vollständigen Einschieben des Fußes der aufgespannte Rand der Einschlüpföffnung des Strumpfes von der abgestuften Kante ablösen kann. Der Kompressionsstrumpf muss bei eingeschobenem menschlichem Fuß nicht mehr an dieser Kante gehalten werden, so dass er mit seiner Dehnung durch den eingeschobenen Fuß im Bereich des Fußgelenks in Zehenrichtung von der Schablone abgezogen und vollständig von der Anziehhilfe getrennt werden kann.

Wahlweise kann die Schablone der Anziehhilfe in den Bereichen, die als Umrisskonturen eines menschlichen Fußes ausgeformt sind, eine im Wesentlichen plattenförmige und flächige oder geringfügig gewölbte Gestalt mit ausgeformten Zehenabschnitten und ausgeformtem Großzehabschnitt aufweisen. Eine solche zweckmäßige Gestaltung kann besonders einfach aus einem flächigen Plattenmaterial gefertigt werden, bspw. aus Kunststoffmaterial oder aus einem Metall, wie bspw. einer Aluminiumlegierung.

Bei einer alternativen Variante der Anziehhilfe kann die Schablone in den Bereichen, die als Umrisskonturen eines menschlichen Fußes ausgeformt sind, zumindest im Bereich der Sohlenfläche eine im Wesentlichen plattenförmige und flächige oder geringfügig gewölbte Gestalt aufweisen, wobei zumindest einer oder einige der ausgeformten Zehenabschnitte und/oder des ausgeformten Großzehabschnittes zumindest abschnittsweise konkav gewölbt sein können.

Die zumindest abschnittsweise konkav gewölbten Zehenabschnitte der Schablone können im Querschnitt oder in Abschnitten ihres jeweiligen Querschnittes jeweils eine U-förmige Kontur aufweisen oder konkav gewölbt sein, so dass trogartige Führungen für die in den Strumpf eingeschobenen Zehen gebildet sind.

Hierbei kann vorgesehen sein, dass die Zehenabschnitte der Schablone zu ihren freien Enden hin flacher gewölbt sind als in ihren der Sohlenfläche näher liegenden Bereichen.

Außerdem kann eine weitere Ausführungsvariante der erfindungsgemäßen Anziehhilfe in einer Weise ausgestaltet sein, dass in den Zehen zugewandten Bereichen der Sohlenfläche der Schablone Erhebungen zur Lenkung der Zehen des in den Strumpf eingeschobenen menschlichen Fußes ausgeformt sind. Diese stegartigen Führungen oder Erhebungen können sich in Bereichen der Schablone befinden, die im fertig eingeschobenen Zustand des Fußes unterhalb der Sohlenoberfläche liegen. Diese stegartigen Führungen können der Lenkung der Zehen bei deren Einschieben in den mittels der Schablone gedehnten Kompressionsstrumpf dienen und können effektiv verhindern, dass ein einzelner Zeh in einen falschen Zehenschlauch, der für einen anderen Zeh vorgesehen ist, eingeschoben wird.

Die erwähnten Erhebungen, die allesamt als optional zu verstehen sind, können insbesondere strahlenförmig zu den Zehen führen, und zwar vorzugsweise so, dass die Längserstreckungsrichtungen der Erhebungen in etwa mit den Längserstreckungsrichtungen der Zehenabschnitte fluchten, und dass die strahlenförmig verlaufenden Erhebungen jeweils in die zwischen benachbarten Zehenabschnitten befindlichen Zwischenräume führen.

Das oben bereits in knapper Form erläuterte Halte-, Fixierungs- und/oder Verankerungselement kann wahlweise mit einem korrespondierenden Gegenlager zusammenwirken und kann dort festlegbar sein, um dem Fuß beim Hineinschlüpfen in den mittels der Schablone gedehnten Kompressionsstrumpf einen ausreichenden Widerstand zu bieten, und um zu verhindern, dass die Schablone mitsamt dem Kompressionsstrumpf dem Fuß ausweicht.

Die Fixierung kann z.B. U-förmig oder L-förmig abgewinkelt gestaltet sein und dient bspw. der Anlage an einer Tischkante, an einer Treppenstufe oder an einem Absatz am Boden. Wenn sich die Schablone in entgegengesetzter Richtung ihrer Zehen im Fersenbereich etwa U-förmig verlängert oder auch nur L-förmig abgewinkelt ist, kann der dadurch gebildete einfache Haken an nahezu jeder Treppenstufe oder Bodenkante eingehängt werden. Der Fuß kann dann bei solchermaßen festgelegter Anziehhilfe in den mittels der Schablone gedehnten und aufgespannten Kompressionsstrumpf eingeschoben werden, ohne dass die Schablone dabei in Einschieberichtung von der den Kompressionsstrumpf anziehenden Person weggeschoben oder weggedrückt wird.

Die Fixierung kann in einer effektiven Ausgestaltung wahlweise auch als Verlängerung des Sohlenbereichs der Schablone in entgegengesetzter Richtung zu den Zehenabschnitten ausgestaltet sein. Die Verlängerung kann insbesondere mit dem Sohlenbereich fluchten und kann dadurch auf dem Boden aufliegen und durch den jeweils nicht in den Kompressionsstrumpf schlüpfenden Fuß fixiert werden, indem sich der Benutzer auf die Verlängerung stellt, während er den anderen Fuß in den auf die Schablone aufgespannten Kompressionsstrumpf hineinschiebt, ohne dass die Schablone dabei in Einschieberichtung von der den Kompressionsstrumpf anziehenden Person weggeschoben oder weggedrückt wird.

Die Schablone der Anziehhilfe kann aus nahezu beliebigen biegesteifen Materialen gefertigt sein, bspw. aus Holz, Metallblech, Kunststoff oder Verbundmaterial. Als Herstellverfahren kann sich bspw. ein Laserschneidverfahren eignen, insbesondere für kleinere Stückzahlen. Ein Stanzverfahren zum Herausstanzen von Metallblechschablonen kann sich für größere Stückzahlen eignen, ebenso ein Spritzgussverfahren für Kunststoffteile.

Sofern die Zehenabschnitte gewölbt sein sollen, kann die Schablone auch folgendermaßen hergestellt sein: Auslasern einer Kunststoffplatte aus thermoplastischem Kunststoffmaterial, Erwärmen bis zur Erweichungstemperatur des thermoplastischen Kunststoffes, Auflegen auf eine Matrize und dadurch ermöglichtes Verrunden der Zehenabschnitte. Denkbar sind auch Laserschneid-, Stanz- und Tiefziehverfahren, ggf. in Kombination.

Vorzugsweise sind die seitlichen Längskanten oder Randkanten der Schablone zur Schonung des gedehnten Strumpfes jeweils verrundet, was außerdem auch dessen Aufziehen und Abziehen erleichtern kann.

Je nach verwendetem Material kann es darüber hinaus sinnvoll sein, die Schablone zu beschichten und/oder zu lackieren, insbesondere um eine glattere Oberfläche zu erhalten, was das Aufziehen und Abziehen des Kompressionsstrumpfes mit seiner normalerweise relativ rauen Oberfläche deutlich erleichtern und damit auch die gesamte Handhabung der Anziehhilfe verbessern kann.

Eine weitere vorteilhafte Ausführungsvariante der erfindungsgemäßen Anziehhilfe kann eine mehrteilige Ausgestaltung vorsehen, bei welcher der wenigstens eine hochgezogene Rand durch ein von der Schablone abnehmbares Seitenteil gebildet ist, das an definierter Position in die Sohlenfläche der Schablone einfügbar oder von dort entnehmbar ist.

Hierbei kann das wenigstens eine den hochgezogenen Rand bildende Seitenteil mittels eines Stecksystems mit der Sohlenfläche der Schablone verbunden werden oder von dort entnommen werden. Das Stecksystem kann insbesondere korrespondierende Ausnehmungen in der Schablone und damit korrespondierende Haken an den Seitenteilen umfassen.

Auf diese Weise kann eine universelle Anziehhilfe zur Verfügung gestellt werden, bei der die Schablone zu beiden Längsseiten jeweils gleichartige Stecksysteme und/oder gleich dimensionierte Ausnehmungen zur Aufnahme der Haken der jeweils dort einfügbaren Seitenteile aufweist. Bei einer solchen Anziehhilfe kann die Schablone vorteilhafterweise beidseitig für den linken oder den rechten Fuß verwendet werden.

Auch wenn dies in der obigen Beschreibung möglicherweise nicht an jeder Stelle deutlich wurde, sei zur Klarstellung an dieser Stelle darauf hingewiesen, dass die erfindungsgemäße Anziehhilfe gleichermaßen für Strümpfe, für Kompressionsstrümpfe, für Unterschenkelstrümpfe wie auch für Kompressions-Unterschenkelstrümpfe und Kompressions-Zehenstrümpfe, aber auch für kurze Kompressionssocken geeignet sein und verwendet werden kann, wobei wahlweise ein von einem Fersenbereich ausgehender Schaftabschnitt verlängert sein kann, wodurch die Anziehhilfe in besonderer Weise auch für über die Unterschenkel reichende Kompressionsstrümpfe geeignet sein kann. Es sei deshalb betont, dass alle oben beschriebenen Ausführungsvarianten der erfindungsgemäßen Anziehhilfe eine solche Verlängerung aufweisen und dadurch für längere Kompressionsstrümpfe oder für Kompressions-Unterschenkelstrümpfe geeignet sein können. Dieser verlängerte Schaftabschnitt kann wahlweise einen gewölbten oder U-förmigen Querschnitt aufweisen, so dass der Strumpf oder Unterschenkelbereich des Strumpfes entsprechend gedehnt wird und eine Öffnung zum Hineinschieben des Fußes und des Unterschenkels gebildet wird.

Somit kann der Unterschenkelstrumpf über eine konkav gewölbte Unterschenkelschablone und die sich einstückig daran anschließende Fußschablone gezogen sein, wodurch sich das Anziehen des Strumpfes kaum von den oben beschriebenen Verfahrensschritten unterscheidet.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Fig. 1 zeigt in zwei schematischen Ansichten (Fig. 1A und Fig. 1B) eine Ausführungsvariante einer erfindungsgemäßen Anziehhilfe zur Ermöglichung oder Erleichterung des Überstreifens eines Strumpfes oder Kompressionsstrumpfes über einen menschlichen Fuß.
Fig. 2 zeigt in insgesamt drei schematischen Ansichten (Fig. 2A, Fig. 2B und Fig. 2C) einer Anziehhilfe gemäß Fig. 1 mit darauf aufgezogenem Kompressions-Zehenstrumpf.
Fig. 3 zeigt in insgesamt elf schematischen Ansichten (Fig. 3A bis Fig. 3K) anhand einer Ausführungsvariante des erfindungsgemäßen Verfahrens die Verwendung einer Anziehhilfe für die Unterstützung beim Überstreifen eines Kompressions-Zehenstrumpfes über einen menschlichen Fuß.
Fig. 4 zeigt in insgesamt vier schematischen Ansichten (Fig. 4A, Fig. 4B, Fig. 4C und Fig. 4D) zwei weitere Ausführungsvarianten einer erfindungsgemäßen Anziehhilfe, deren Einzelteile ineinander gefügt werden können.
Fig. 5 zeigt in insgesamt zehn schematischen Ansichten (Fig. 5A bis Fig. 5J) aufeinander folgende Prozessschritte bei der Verwendung der in den Figuren 4C und 4D gezeigten Variante der Anziehhilfe im Zusammenhang mit dem Überziehen eines Kompressionsstrumpfes über einen menschlichen Fuß.
Fig. 6 zeigt in insgesamt sieben schematischen Ansichten (Fig. 6A bis Fig. 6G) weitere aufeinander folgende Prozessschritte bei der Verwendung der in den Figuren 4C und 4D gezeigten Variante der Anziehhilfe zum Überziehen eines Kompressionsstrumpfes über einen menschlichen Fuß.

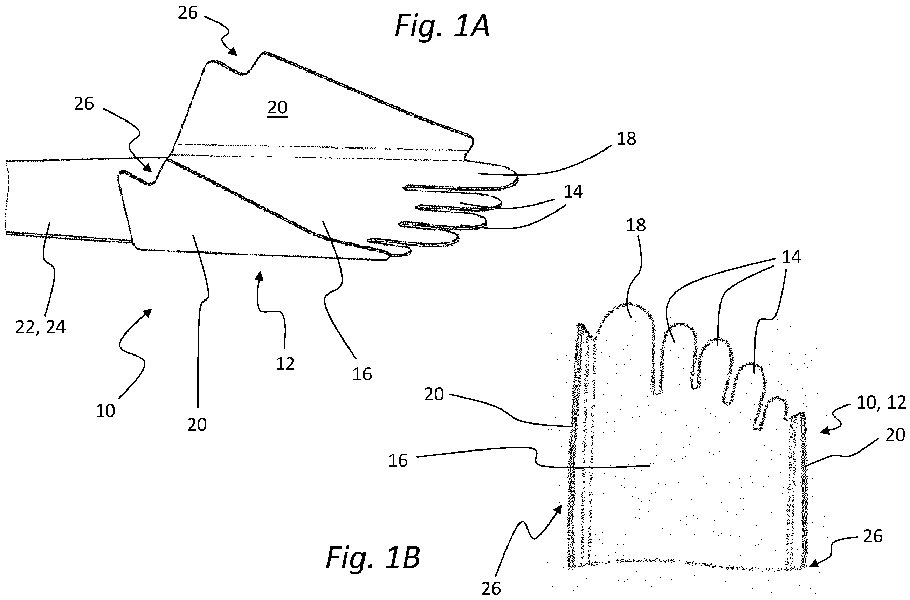

Für gleiche oder gleich wirkende Elemente der Erfindung werden in den Figuren 1A bis 6G jeweils gleiche Bezugsziffern verwendet. Ferner werden der Übersicht halber nur solche Bezugszeichen in den einzelnen Figuren dargestellt, die für die Beschreibung der jeweiligen Figur erforderlich sind. Die dargestellten Ausführungsformen stellen lediglich Beispiele dar, wie die Erfindung ausgestaltet sein kann und stellen keine abschließende Begrenzung dar. Auch sind die nachfolgend beschriebenen Merkmale jeweils nicht unbedingt in einem engen Zusammenhang mit weiteren Merkmalen des jeweiligen Ausführungsbeispiels zu verstehen, sondern können jeweils im allgemeinen Zusammenhang vorgesehen sein bzw. hierfür Verwendung finden.

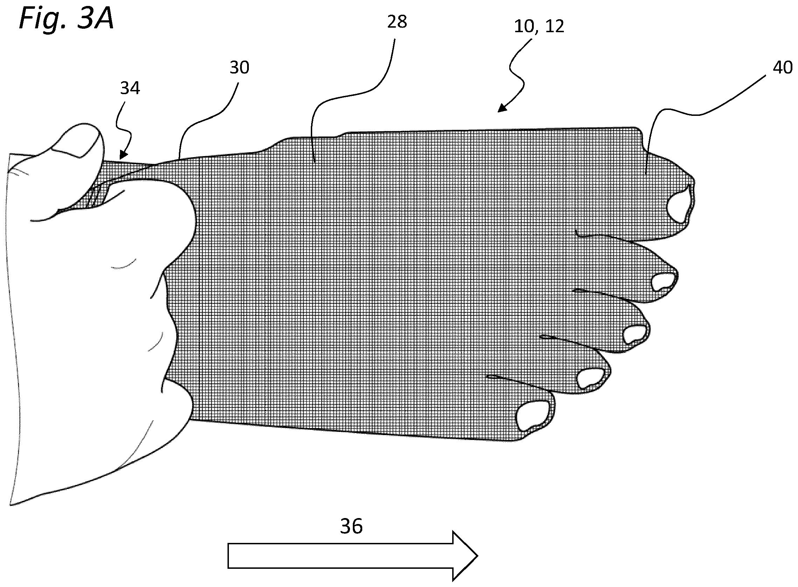

Die Fig. 1 zeigt in zwei schematischen Ansichten (Fig. 1A und Fig. 1B) eine Ausführungsvariante einer erfindungsgemäßen Anziehhilfe 10 zur Ermöglichung oder Erleichterung des Überstreifens eines Strumpfes über einen menschlichen Fuß (vgl. hierzu die schematischen Darstellungen der Figuren 3A bis 3K). Bei dem überzustreifenden oder anzuziehenden Strumpf, der in den Figuren 1A und 1B nicht gezeigt ist, kann es sich insbesondere um einen Kompressionsstrumpf oder Kompressions-Zehenstrumpf handeln, der im entspannten Zustand ohne darin aufgenommenen Fuß zumindest abschnittsweise ein kleineres Volumen einnimmt als im gedehnten, über den Fuß gezogenen Zustand.

Die in der Fig. 1A in schematischer und perspektivischer Seitenansicht und in Fig. 1B in perspektivischer Draufsicht gezeigte Ausführungsvariante der Anziehhilfe 10 umfasst eine aus widerstandsfähigem und zumindest weitgehend formstabilem Flachmaterial wie etwa aus Aluminiumblech gefertigte Schablone 12 mit Umrisskonturen, die zumindest bereichsweise einem ausgestreckten und/oder flach auf einer Unterlage stehenden menschlichen Fuß ähneln. Die in ihrer Umrisskontur der Größe eines menschlichen Fußes entsprechende Schablone 12 weist leicht voneinander abgespreizte und seitlich voneinander beabstandete Zehenabschnitte 14 sowie einen von der Sohlenfläche 16 der Schablone 12 abstehenden etwas größeren Großzehabschnitt 18 auf.

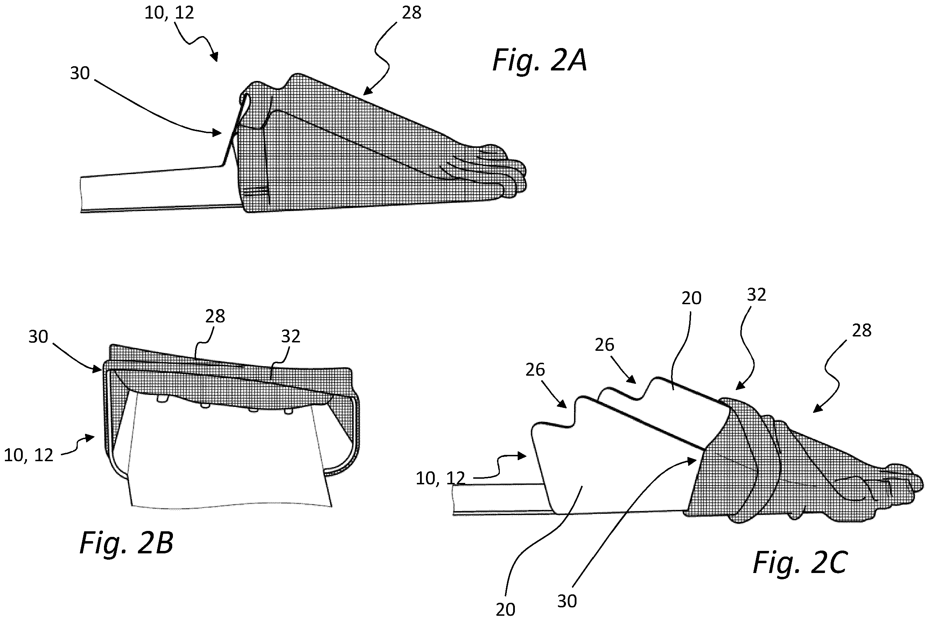

Weiterhin umfasst die Schablone 12 an beiden Längsseiten der Sohlenfläche 16 jeweils hochgezogene Ränder 20, deren Formgebung bereits aus den Figuren 1A und 1B deutlich wird, während ihre Aufgabe und Funktionsweise anhand der Figuren 2A bis 2C sowie insbesondere anhand der Figuren 3A bis 3K näher erläutert wird.

Weiterhin umfasst die Schablone 12 ein Verankerungselement 22 zur zumindest temporären Fixierung oder Abstützung der Schablone 12 beim Einführen des mit einem Strumpf zu bedeckenden Fußes gegen elastische Rückstellkräfte des über die Schablone 12 gezogenen und dabei entsprechend den Umrisskonturen der Schablone 12 gedehnten Strumpfes.

Das Verankerungselement 22 ist im dargestellten Ausführungsbeispiel durch einen sich rückseitig am Fersenbereich an die Sohlenfläche 16 anschließenden Blechabschnitt 24 gebildet, der somit eine Verlängerung des sich an den Bereich der Sohlenfläche 16 der Schablone 12 anschließenden Schablonenmaterials bildet. Der verlängerte Blechabschnitt 24 des Verankerungselements 22 kann bspw. auf eine Unterlage bzw. auf den Boden gelegt und mit dem zweiten Fuß fixiert werden, wenn der Benutzer mit dem anderen Fuß in einen auf die Schablone 12 aufgezogenen Strumpf schlüpft. Wie es die Fig. 1A erkennen lässt, befindet sich das Verankerungselement 22 an der den Zehenabschnitten 14 und 18 gegenüberliegenden Rückseite der Schablone 12.

Die an beiden Längsseiten der Sohlenfläche 16 der Schablone 12 hochgezogenen Ränder 20 sind in Richtung des Verankerungselements 22 höher gezogen als in einer Richtung zu den Zehenabschnitten 14 und 18, wodurch eine Öffnung des auf die Schablone 12 aufgezogenen Kompressionsstrumpfes, die etwa im Übergangsbereich zum Verankerungselement 22 liegen kann, weiter geöffnet ist als die Bereiche des Kompressionsstrumpfes, die näher an den Zehenabschnitten 14 und 18 liegen, so dass der Fuß in die Strumpföffnung leichter hineingeführt werden kann.

Wie es insbesondere die perspektivische Seitenansicht der Fig. 1A, aber auch die perspektivische Draufsicht der Fig. 1B erkennen lassen, weisen die hochgezogenen Ränder 20 jeweils keil- oder dreieckförmige Konturen auf, deren kurze Seiten im Bereich des Mittelfußes oder näher zur Ferse liegen, ungefähr rechtwinkelig zur Sohlenfläche 16 stehen. Die sich an die kurzen Seiten jeweils anschließenden langen Seiten der Keile oder Dreiecke laufen in einem Winkel (bspw. ca. 25 - 40°) in Richtung zu einem randseitigen Großzehabschnitt 18 bzw. zum Zehenabschnitt 14 für den kleinen Zeh hin aus und reichen in den Zehenabschnitt 14 für den kleinen Zeh bzw. in den Großzehabschnitt 18 hinein.

Die hochgezogenen Ränder 20 sind nicht scharf abgekantet, sondern in einem definierten Radius umgebogen und weisen in etwa rechtwinkelig von der Sohlenfläche 16 ausgehend nach oben, so dass die durch die hochgezogenen Ränder 20 aufgespannten Ebenen in etwa parallel zueinander orientiert sein können (vgl. Fig. 1B). Die Fig. 1B lässt allerdings minimal oder leicht wannenartig nach außen geneigte Ränder 20 erkennen, deren obere Kanten geringfügig weiter voneinander entfernt sind als ihre unteren Abschnitte, die an die Sohlenfläche 16 der Schablone 12 anschließen.

Die freien Enden der Zehenabschnitte 14 und des Großzehabschnittes 18 weisen stirnseitig jeweils einen Halbkreisradius mit einem Durchmesser auf, der in etwa der Breite des jeweiligen Zehenabschnittes 14 bzw. des Großzehabschnittes 18 entspricht. Eine solche Konturierung ist deshalb sinnvoll, weil auch die geschlossenen Enden der Zehenschläuche üblicher Strümpfe eine gewölbte Kontur aufweisen, so dass eine mit Rundung versehene Konturierung der Schablone 12 der Formgebung der Strümpfe entspricht.

Die hochgezogenen Ränder 20 sind an der oberen Kante der im mittleren Bereich der Sohle (eines hier nicht dargestellten Fußes) befindlichen kurzen Seite abgestuft ausgebildet, wodurch der Eckbereich im Übergang zur langen keilförmigen Seite des Randes 20 jeweils eine stufenartige Aussparung 26 bildet. Eine solche Abstufung 26 in der Höhe beider Ränder 20 kann helfen, dass der Kompressionsstrumpf zunächst an seinem Platz gehalten wird und nicht nach vorne in Richtung der Zehen abrutscht, wenn zunächst erst die Zehen in die Strumpföffnung eingeschoben werden. Dieser Zusammenhang wird anhand der Figuren 3A ff. verdeutlicht.

Die insgesamt drei schematischen Ansichten der Figuren 2A, 2B und 2C verdeutlichen die Schablone 12 der Anziehhilfe 10 gemäß Fig. 1A und Fig. 1B mit darauf aufgezogenem Kompressionsstrumpf 28. Dort sind nochmals die abgestuften oberen Kanten der hochgezogenen Ränder 20 der Schablone 12 erkennbar. An den dort ausgeformten Abstufungen 26 in den Eckbereichen im Übergang der kurzen Seite des jeweiligen Randes 20 zur langen keilförmigen Seite des Randes 20 kann der Kompressionsstrumpf 28 in der in den Figuren 2A und 2B gezeigten Weise zunächst an seinem Platz gehalten werden, so dass er nicht nach vorne in Richtung der Zehen abrutscht, wenn zunächst erst die Zehen in die Strumpföffnung 30 eingeschoben werden.

Die schematische Seitenansicht der Fig. 2C verdeutlicht eine Situation eines mit seinen Zehen teilweise durch die Strumpföffnung 30 in den Kompressionsstrumpf 28 eingeschobenen menschlichen Fußes (nicht gezeigt; vgl. hierzu die Figuren 3A ff.). Beim weiteren Einschieben des Fußes wölbt die ein größeres Volumen beanspruchende Spannbereich mit dem oftmals voluminöseren Mittelfußbereich die Einschlüpföffnung 30 des Strumpfes 28 stärker nach oben, d.h. in einer Richtung senkrecht zur Oberfläche der Schablone 12, die sich zwischen den seitlichen hochgezogenen Rändern 20 befindet, bis sich schließlich beim weiteren oder vollständigen Einschieben des Fußes der aufgespannte Rand 32 der Einschlüpföffnung 30 des Strumpfes 28 von der abgestuften Kante 26 ablösen kann.

Der Kompressionsstrumpf 28 muss bei eingeschobenem menschlichen Fuß nicht mehr an dieser Abstufung 26 gehalten werden, so dass er mit seiner Dehnung durch den eingeschobenen Fuß im Mittelfußbereich in Zehenrichtung von der Schablone 12 abgezogen und nachfolgend vollständig von der Anziehhilfe 10 getrennt werden kann (nicht gezeigt in Fig. 2C).

Die insgesamt elf schematischen Ansichten der Figuren 3A bis 3K zeigen anhand einer Ausführungsvariante des erfindungsgemäßen Verfahrens die Verwendung einer Anziehhilfe 10 gemäß Figuren 1A bis 2C als Unterstützung beim Überstreifen eines Kompressionsstrumpfes 28 über einen menschlichen Fuß 34. Dargestellt sind aufeinanderfolgende Prozessphasen beim Überstreifen eines Kompressionsstrumpfes 28 - hier dargestellt anhand eines Kompressions-Zehenstrumpfes 28 - über einen rechten Fuß 34.

So zeigt die schematische Draufsicht der Fig. 3A einen gemäß Fig. 2A und Fig. 2B über eine Schablone 12 einer Anziehhilfe 10 gezogenen Kompressions-Zehenstrumpf 28, durch dessen aufgespannte und an den stufenartigen Aussparungen 26 der hochgezogenen Ränder 20 der Schablone 12 eingehängte Einschlüpföffnung 30 der rechte Fuß 34 des zukünftigen Trägers des Strumpfes 28 bereits teilweise hineingeschoben ist. Es muss nicht erwähnt werden, dass die Bewegungsrichtung 36 hierbei von links nach rechts erfolgt. Zur Unterstützung kann die Einschlüpföffnung 30 des Strumpfes 28 dabei händisch festgehalten werden (vgl. Fig. 3A).

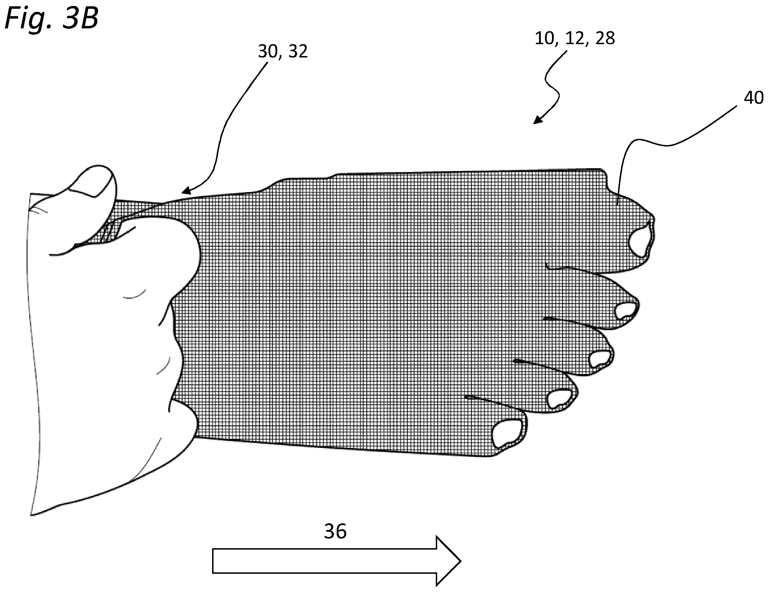

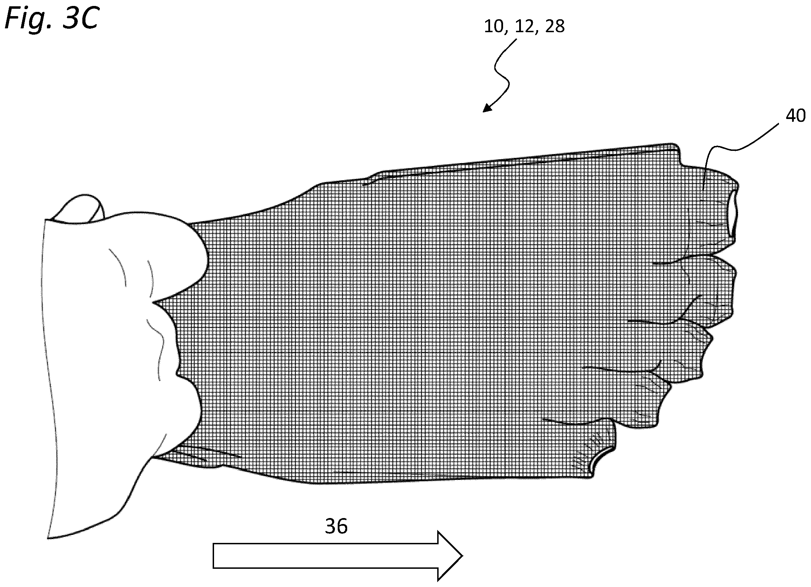

Bei der Draufsicht der Fig. 3B sind die Zehen des Fußes 34 bereits etwas weiter in den Kompressionsstrumpf 28 eingetaucht und in der in Fig. 3C gezeigten Prozessphase noch weiter durch die Einschlüpföffnung 30 in den Strumpf 28 hineingeschoben, so dass sich hier bereits der Strumpf 28 an seiner Rückseite nach oben wölbt.

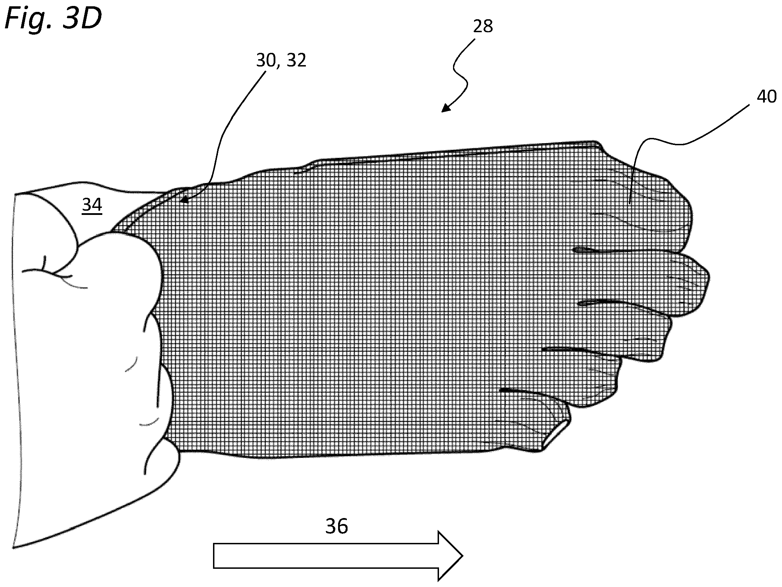

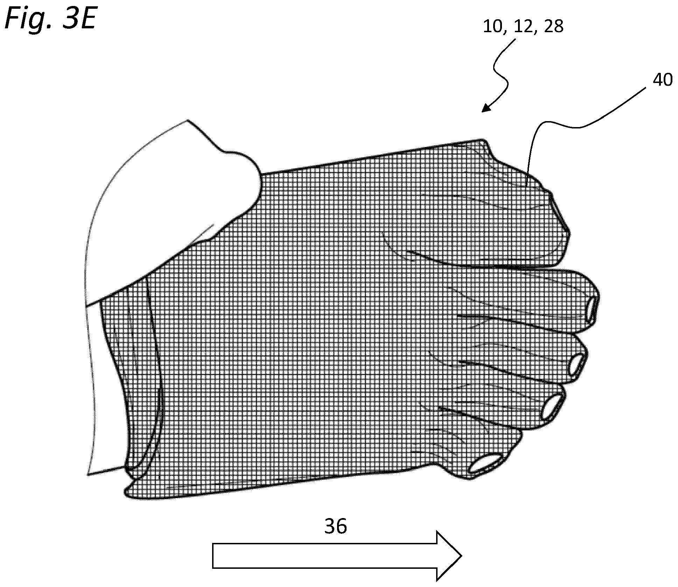

Sobald die Zehen des in den Kompressionsstrumpf 28 eingeschobenen menschlichen Fußes 34 die Zehenschläuche 40 des Strumpfes 28 erreichen, wölbt der ein größeres Volumen beanspruchende Mittelfußbereich die Einschlüpföffnung 30 des Strumpfes 28 stärker nach oben, d.h. in einer Richtung senkrecht zur Oberfläche der Schablone 12, die sich zwischen den seitlichen hochgezogenen Rändern 20 befindet (vgl. Fig. 3D und Fig. 3E), bis sich schließlich beim noch weiteren Einschieben des Fußes 34 der aufgespannte Rand 32 der Einschlüpföffnung 30 des Strumpfes 28 von den abgestuften Kanten 26 der hochgezogenen Ränder 20 der Schablone 12 ablösen kann.

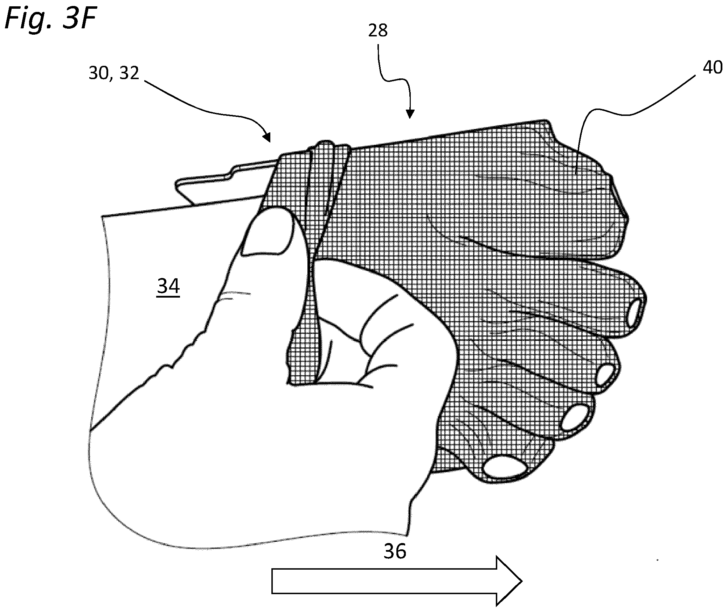

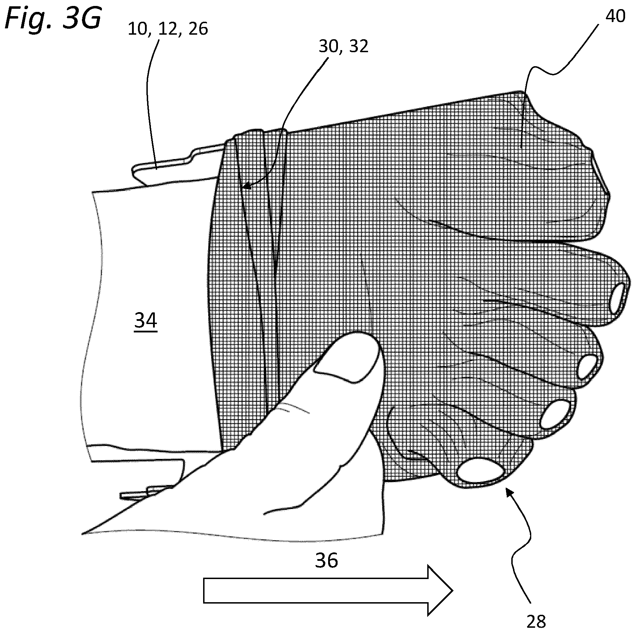

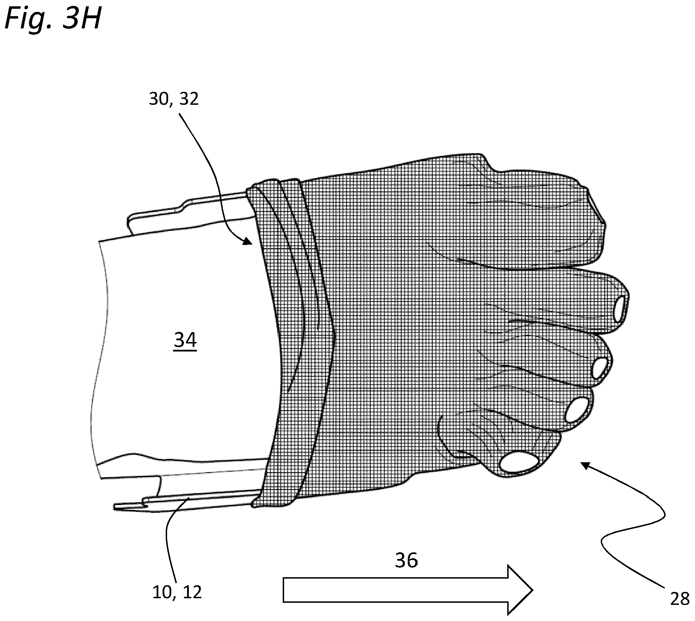

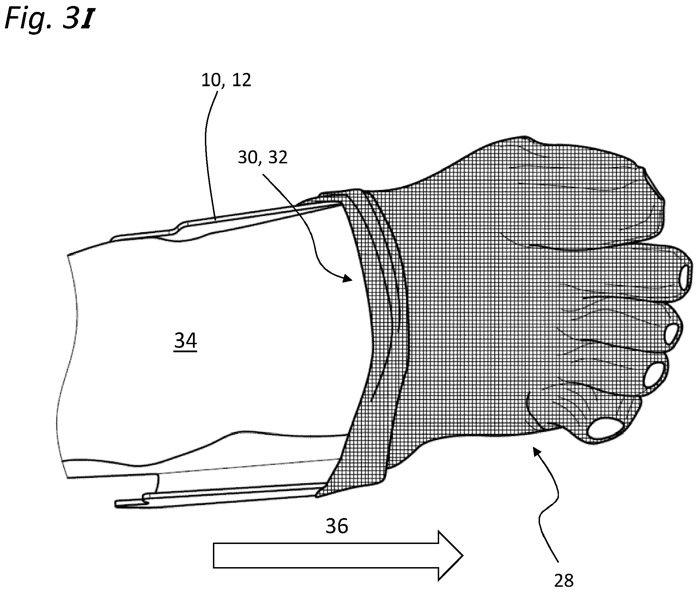

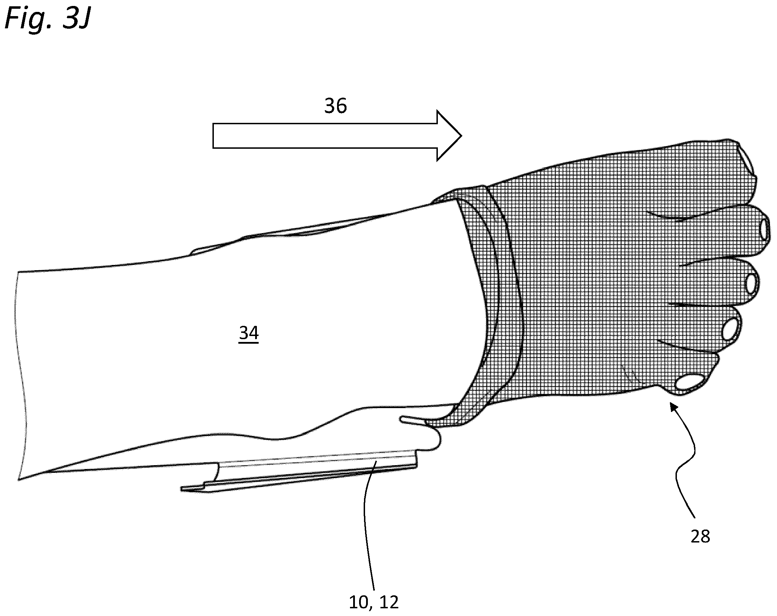

Wie es die Figuren 3F und 3G zeigen, muss der Kompressionsstrumpf 28 bei eingeschobenem menschlichem Fuß 34 nicht mehr an dieser Kante 26 gehalten werden, so dass er mit seiner Dehnung durch den eingeschobenen Fuß 34 in Zehenrichtung 36 zunächst durch manuelle Unterstützung von den beiden Kanten 26 abgehoben (vgl. Fig. 3F und Fig. 3G) und anschließend mit entlang der Ränder 20 herabrutschendem Rand 32 des Strumpfes 28 (vgl. Fig. 3H und Fig. 3I) Stück für Stück von der Anziehhilfe 10 getrennt werden kann (vgl. Fig. 3J).

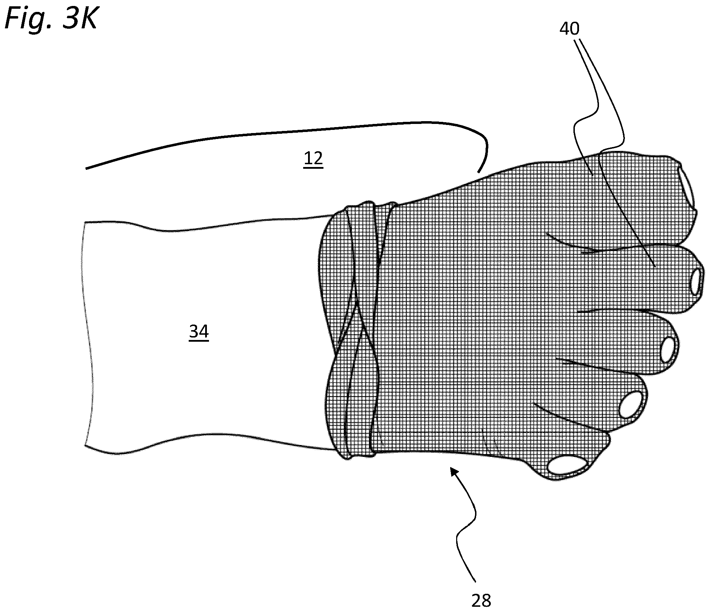

Die letzte Fig. 3K zeigt den mit dem Strumpf 28 ausgestatteten rechten Fuß 34, der vollständig von der Anziehhilfe 10 getrennt ist. Der Strumpf 28 bedeckt den Fuß 34 allerdings noch nicht vollständig, sondern muss nun über die Ferse und das Fußgelenk gezogen werden, was jedoch viel leichter gelingen kann als beim Überstreifen ohne eine solche Anziehhilfe 12.

Rein vorsorglich sei darauf hingewiesen, dass bei den in den Figuren 2A bis 3K abgebildeten Kompressionsstrümpfen 28 zwar die Zehenspitzen an den freien Enden der Zehenschläuche 40 fehlen. Eine solche Ausführungsoption kann sinnvoll sein, da die vordersten Zehenspitzen einer Kompressionswirkung durch die Strümpfe 28 nicht zugänglich sind. Es handelt sich bei dem Strumpf somit um einen sog. Kompressions-Zehenstrumpf 28.

Ebenso können die Zehenschläuche 40 auch stirnseitig geschlossen sein (hier nicht gezeigt).

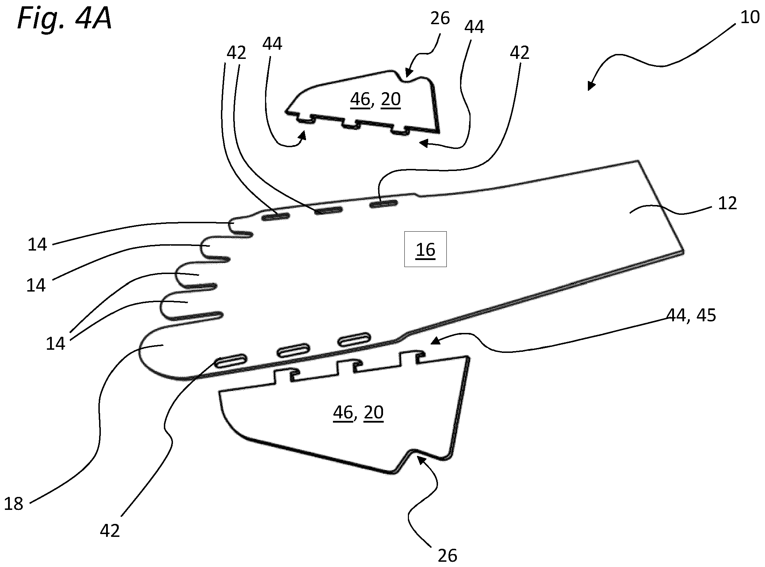

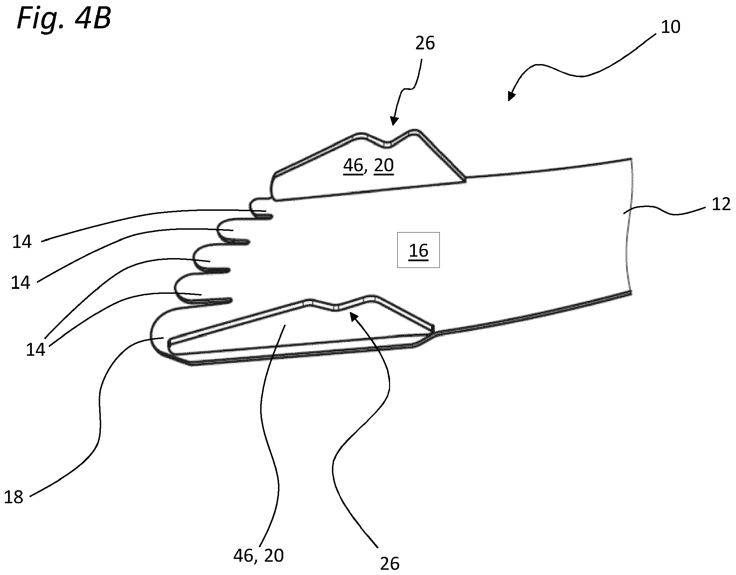

Die Figuren 4A und 4B zeigen in zwei schematischen Ansichten eine weitere Ausführungsvariante einer erfindungsgemäßen Anziehhilfe, deren Einzelteile aneinander und ineinander gefügt werden können.

So zeigt die Fig. 4A eine durch drei Einzelteile gebildete Anziehhilfe, die gemäß der Darstellung der Fig. 4B ineinandergesteckt und solchermaßen zusammengefügt werden können.

Da die Anziehhilfe 10 in dem in Fig. 4A gezeigten zerlegten Zustand noch nicht als solche funktioniert, soll hier zunächst die Schablone 12 erläutert werden. Wie bei den zuvor erläuterten Ausführungsvarianten umfasst auch die hier erläuterte mehrteilige Anziehhilfe eine aus widerstandsfähigem und zumindest weitgehend formstabilem Flachmaterial wie etwa aus einem thermoplastischen Kunststoff oder aus Aluminiumblech gefertigte Schablone 12 mit Umrisskonturen, die zumindest bereichsweise einem ausgestreckten und/oder flach auf einer Unterlage stehenden menschlichen Fuß ähneln. Die in ihrer Umrisskontur der Größe eines menschlichen Fußes entsprechende Schablone 12 weist leicht voneinander abgespreizte und seitlich voneinander beabstandete Zehenabschnitte 14 sowie einen von der Sohlenfläche 16 der Schablone 12 abstehenden etwas größeren Großzehabschnitt 18 auf.

Weiterhin umfasst die Schablone 12 an beiden Längsseiten der Sohlenfläche 16 jeweils drei längliche Ausnehmungen 42 auf, die in Längserstreckungsrichtung jeweils miteinander fluchten und ungefähr gleiche Abstände voneinander aufweisen. Die an jeder Seite in etwa im Ballenbereich des Fußes (nicht gezeigt) befindlichen drei hintereinander angeordneten Ausnehmungen 42 befinden sich unmittelbar am linken und am rechten Rand der Sohlenfläche 16.

Jede der länglichen Ausnehmungen 42 durchdringt die Sohlenfläche 16 und wirkt je nach Zusammenbauzustand mit korrespondierenden Haken 44 zusammen, die sich an unteren Stirnseiten zweier Randabschnitte 46 oder Seitenteile 46 befinden und die im zusammengebauten Zustand der Anziehhilfe 10 (Fig. 4B) in die länglichen Ausnehmungen 42 eingefügt und dort verhakt werden können.

Die mit den länglichen Ausnehmungen 42 zusammenwirkenden Haken 44 werden im vorliegenden Zusammenhang auch als Stecksystem 45 bezeichnet.

In diesem in Fig. 4B gezeigten Zusammenbauzustand der dreiteiligen Anziehhilfe 10 fungieren die Randabschnitte 46 oder Seitenteile 46 als hochgezogene Ränder 20, deren Formgebung bereits zuvor anhand der Figuren 1A und 1B verdeutlich wurde, während ihre Aufgabe und Funktionsweise anhand der Figuren 2A bis 2C sowie anhand der Figuren 3A bis 3K näher erläutert wurde.

Wie bereits zuvor erläutert, sind die an beiden Längsseiten der Sohlenfläche 16 der Schablone 12 hochgezogenen Ränder 20 in Richtung entgegengesetzt zu den Zehenabschnitten 14 und 18 höher gezogen als in einer Richtung zu den Zehenabschnitten 14 und 18, wodurch eine Öffnung des auf die Schablone 12 aufgezogenen Kompressionsstrumpfes weiter geöffnet ist als die vorderen Bereiche des Kompressionsstrumpfes, die näher an den Zehenabschnitten 14 und 18 liegen, so dass der Fuß in die Strumpföffnung leichter hineingeführt werden kann.

Die perspektivischen Ansichten der Figuren 4A und 4B lassen gleichermaßen erkennen, dass die durch die in die länglichen Ausnehmungen 42 an den Längsrändern der Sohlenfläche 16 einsteckbaren Randabschnitte 46 (auch als Seitenteile 46 bezeichnet) bzw. die dort eingesteckten hochgezogenen Ränder 20 jeweils keil- oder dreieckförmige Konturen aufweisen, deren kurze Seiten im Bereich des Mittelfußes oder näher zur Ferse liegen.

Außerdem lässt die Fig. 4B erkennen, dass die Seitenteile 46 oder Randabschnitte 46 im in die Sohlenfläche 16 eingefügten Zustand ungefähr rechtwinkelig zur Sohlenfläche 16 stehen. Die sich an die kurzen Seiten jeweils anschließenden langen Seiten der Keile oder Dreiecke laufen in einem Winkel (bspw. ca. 25 - 40°) in Richtung zu einem randseitigen Großzehabschnitt 18 bzw. zum Zehenabschnitt 14 für den kleinen Zeh hin aus und reichen in den Zehenabschnitt 14 für den kleinen Zeh bzw. in den Großzehabschnitt 18 hinein.

Wie schon zuvor erläutert, weisen auch bei der hier gezeigten Anziehhilfe 10 die freien Enden der Zehenabschnitte 14 und des Großzehabschnittes 18 stirnseitig jeweils einen Halbkreisradius mit einem Durchmesser auf, der in etwa der Breite des jeweiligen Zehenabschnittes 14 bzw. des Großzehabschnittes 18 entspricht.

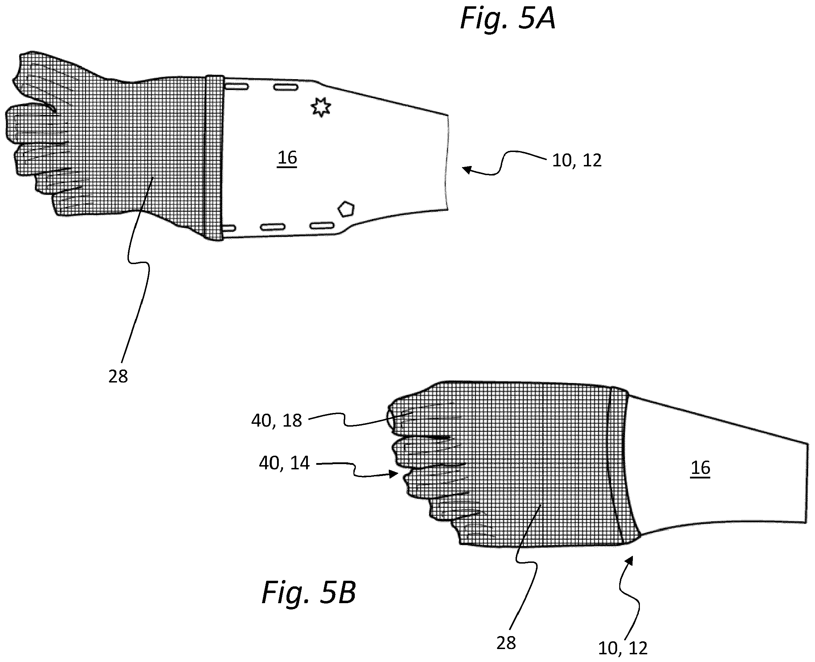

Die durch die einsteckbaren Seitenteile oder Randabschnitte 46 gebildeten hochgezogenen Ränder 20 sind an der oberen Kante der im mittleren Bereich der Sohle (eines hier nicht dargestellten Fußes) befindlichen kurzen Seite abgestuft ausgebildet, wodurch der Eckbereich im Übergang zur langen keilförmigen Seite des Randes 20 jeweils eine stufenartige Aussparung 26 bildet. Eine solche Abstufung 26 in der Höhe beider Ränder 20 oder Randabschnitte 46 kann helfen, dass der Kompressionsstrumpf zunächst an seinem Platz gehalten wird und nicht nach vorne in Richtung der Zehen abrutscht, wenn zunächst erst die Zehen in die Strumpföffnung eingeschoben werden. Dieser Zusammenhang wurde bereits anhand der Figuren 3A ff. verdeutlicht und wird nochmals anhand der Figuren 5A bis 6G erläutert.

Wie es die Fig. 4A erkennen lässt, sind die drei hintereinander an den jeweiligen unteren Rändern der Randabschnitte 46 angeordneten Haken 44 so dimensioniert, dass sie sich mit geringem Spiel in die länglichen Ausnehmungen 42 einfügen und durch Verschieben der Randabschnitte 46 oder Seitenteile 46 parallel zur Längserstreckungsrichtung der Sohlenfläche 16 in einer Richtung entgegengesetzt zu den Zehenabschnitten 14 und 18 verhaken lassen.

Außerdem lässt die Fig. 4A die besondere Form der Haken 44 erkennen, die jeweils als Widerhaken ausgebildet sind und ein senkrechtes Einschieben der Randabschnitte 46 oder Seitenteile 46 von oben in die länglichen Ausnehmungen 42, d.h. senkrecht zur Oberfläche der Sohlenfläche, erlauben, bis die unteren Ränder der Randabschnitte 46 oder Seitenteile 46 auf der Sohlenfläche 16 aufliegen und die Haken 44 die Ausnehmungen 42 durchdringen. Ein anschließendes Verschieben der Seitenteile oder Randabschnitte 46 in einer Richtung entgegengesetzt zu den Zehenabschnitten 14 und 18 verhakt die als Widerhaken ausgebildeten Haken 44 und damit die Seitenteile oder Randabschnitte 46 im Sohlenabschnitt 16 und verhindert ein einfaches Abziehen nach oben.

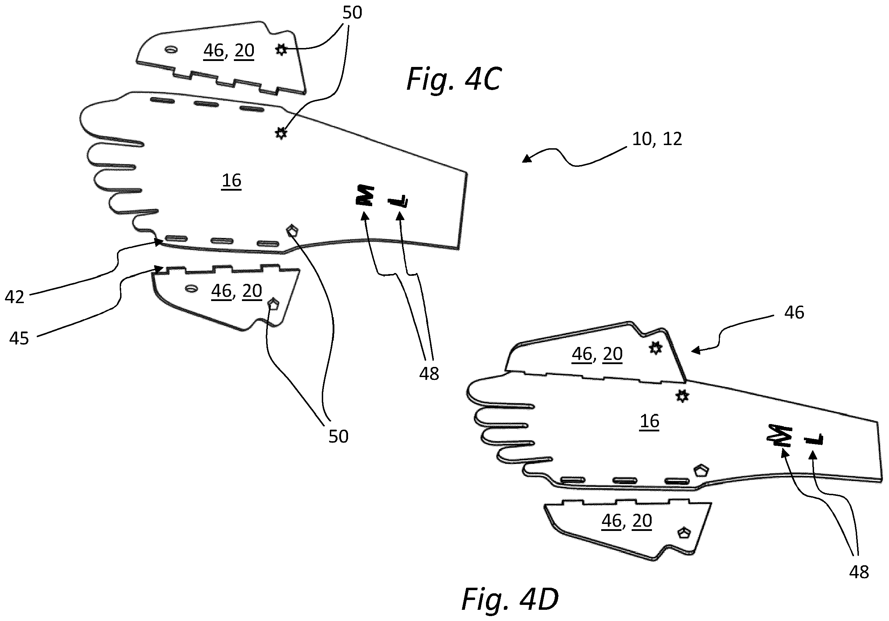

Die Figuren 4C und 4D zeigen in zwei schematischen Ansichten eine leicht abgewandelte weitere Ausführungsvariante einer erfindungsgemäßen Anziehhilfe, deren Einzelteile aneinander und ineinander gefügt werden können. So zeigt die Fig. 4C eine durch drei Einzelteile gebildete Anziehhilfe, die gemäß der Darstellung der Fig. 4D ineinandergesteckt und solchermaßen zusammengefügt werden können.

Die Anziehhilfe 10 gemäß Figuren 4C und 4D unterscheidet sich nur in kleineren Details von der in den in Fig. 4A und in Fig. 4B gezeigten Variante. Wie diese Variante umfasst auch die in den Figuren 4C und 4D gezeigte Variante der Schablone 12 an beiden Längsseiten der Sohlenfläche 16 jeweils drei längliche Ausnehmungen 42, die in Längserstreckungsrichtung jeweils miteinander fluchten und ungefähr gleiche Abstände voneinander aufweisen. Die an jeder Seite in etwa im Ballenbereich des Fußes (nicht gezeigt) befindlichen drei hintereinander angeordneten Ausnehmungen 42 befinden sich unmittelbar am linken und am rechten Rand der Sohlenfläche 16.

Jede der länglichen Ausnehmungen 42 durchdringt die Sohlenfläche 16 und wirkt je nach Zusammenbauzustand mit korrespondierenden Haken 44 zusammen, die sich an unteren Stirnseiten zweier Randabschnitte 46 oder Seitenteile 46 befinden und die im zusammengebauten Zustand der Anziehhilfe 10 (Fig. 4B) in die länglichen Ausnehmungen 42 eingefügt und dort verhakt werden können.

Auch in dem in Fig. 4D gezeigten Zusammenbauzustand der dreiteiligen Anziehhilfe 10 fungieren die Randabschnitte 46 oder Seitenteile 46 als hochgezogene Ränder 20, deren Formgebung bereits zuvor anhand der Figuren 1A, 1B sowie 4A und 4B verdeutlich wurde, während ihre Aufgabe und Funktionsweise anhand der Figuren 2A bis 2C sowie anhand der Figuren 3A bis 3K näher erläutert wurde.

Wie bereits zuvor erläutert, sind die an beiden Längsseiten der Sohlenfläche 16 der Schablone 12 hochgezogenen Ränder 20 in Richtung entgegengesetzt zu den Zehenabschnitten 14 und 18 höher gezogen als in einer Richtung zu den Zehenabschnitten 14 und 18, wodurch eine Öffnung des auf die Schablone 12 aufgezogenen Kompressionsstrumpfes weiter geöffnet ist als die vorderen Bereiche des Kompressionsstrumpfes, die näher an den Zehenabschnitten 14 und 18 liegen, so dass der Fuß in die Strumpföffnung leichter hineingeführt werden kann.

Die perspektivischen Ansichten der Figuren 4C und 4D lassen gleichermaßen erkennen, dass die durch die in die länglichen Ausnehmungen 42 an den Längsrändern der Sohlenfläche 16 einsteckbaren Randabschnitte 46 (auch als Seitenteile 46 bezeichnet) bzw. die dort eingesteckten hochgezogenen Ränder 20 jeweils keil- oder dreieckförmige Konturen aufweisen, deren kurze Seiten im Bereich des Mittelfußes oder näher zur Ferse liegen.

Außerdem lässt die Fig. 4D erkennen, dass die Seitenteile 46 oder Randabschnitte 46 im in die Sohlenfläche 16 eingefügten Zustand ungefähr rechtwinkelig zur Sohlenfläche 16 stehen. Die sich an die kurzen Seiten jeweils anschließenden langen Seiten der Keile oder Dreiecke laufen in einem Winkel (bspw. ca. 25 - 40°) in Richtung zu einem randseitigen Großzehabschnitt 18 bzw. zum Zehenabschnitt 14 für den kleinen Zeh hin aus und reichen in den Zehenabschnitt 14 für den kleinen Zeh bzw. in den Großzehabschnitt 18 hinein.

Wie schon zuvor erläutert, weisen auch bei der hier gezeigten Anziehhilfe 10 die freien Enden der Zehenabschnitte 14 und des Großzehabschnittes 18 stirnseitig jeweils einen Halbkreisradius mit einem Durchmesser auf, der in etwa der Breite des jeweiligen Zehenabschnittes 14 bzw. des Großzehabschnittes 18 entspricht.

Die durch die einsteckbaren Seitenteile oder Randabschnitte 46 gebildeten hochgezogenen Ränder 20 sind an der oberen Kante der im mittleren Bereich der Sohle (eines hier nicht dargestellten Fußes) befindlichen kurzen Seite abgestuft ausgebildet, wodurch der Eckbereich im Übergang zur langen keilförmigen Seite des Randes 20 jeweils eine stufenartige Aussparung 26 bildet. Eine solche Abstufung 26 in der Höhe beider Ränder 20 oder Randabschnitte 46 kann helfen, dass der Kompressionsstrumpf zunächst an seinem Platz gehalten wird und nicht nach vorne in Richtung der Zehen abrutscht, wenn zunächst erst die Zehen in die Strumpföffnung eingeschoben werden. Dieser Zusammenhang wurde bereits anhand der Figuren 3A ff. verdeutlicht und wird nochmals anhand der Figuren 5A bis 6G erläutert.

Wie es die Fig. 4C erkennen lässt, sind die drei hintereinander an den jeweiligen unteren Rändern der Randabschnitte oder Seitenteile 46 angeordneten Haken 44 so dimensioniert, dass sie sich mit geringem Spiel in die länglichen Ausnehmungen 42 einfügen lassen (vgl. Fig. 4D).

Während die Haken 44 bei der Ausführungsvariante gemäß Fig. 4A jeweils als Widerhaken ausgebildet sind, sind die Haken 44 der Variante gemäß Fig. 4C und Fig. 4D jeweils rechteckförmig und ohne Widerhaken ausgebildet. Die Seitenteile 46 können somit einfach nur von oben die Sohlenfläche 16 eingesteckt werden und sind nur dann etwas starrer verankert, wenn die Haken 44 sehr eng und spielfrei in den länglichen Ausnehmungen 42 sitzen. Wenn dies nicht der Fall ist und ein geringes Spiel verbleibt, ist die Funktion der mehrteiligen Anziehhilfe gleichwohl nicht beeinträchtigt, denn die Seitenteile 46 oder Randabschnitte werden durch den Kompressionsstrumpf ohnehin in ihrer in Fig. 4D gezeigten Lage gehalten, sobald sie in die Ausnehmungen 42 eingesteckt wurden.

Die Fig. 4D zeigt ein eingestecktes Seitenteil 46 am Innenfußbereich, d.h. am Rand der Sohlenfläche 16, die zum Großzehabschnitt 18 reicht, während das andere Seitenteil 46 neben der Sohlenfläche 16 liegt.

Ein besonderer Vorteil der in den Figuren 4A bis 4D gezeigten Varianten der mehrteiligen und zusammenbaubaren Anziehhilfe 10 besteht darin, dass die Sohlenfläche 16 nicht mehr für einen linken und für einen rechten Fuß gefertigt werden muss, sondern je nach Bedarf einfach umgedreht werden kann. Da die länglichen Ausnehmungen 42 und die korrespondierenden Haken 44 der Seitenteile 46 wahlweise von beiden Oberflächenrichtungen der Sohlenfläche 16 eingesteckt und zusammengefügt werden können, kann die Anziehhilfe 10 für beide Füße gleichermaßen verwendet werden. Die Sohlenfläche 16 weist hierzu auf beiden Oberflächen jeweils Markierungen 48 für den linken Fuß bzw. für den rechten Fuß auf, die bspw. durch eingeprägte oder erhabene Großbuchstaben "L" (links; siehe Fig. 4C und Fig. 4D) oder "R" (rechts; nicht gezeigt) gebildet sein können.

Eine weitere Markierung 48 kann bspw. eine Größenangabe sein, wie hier durch den Großbuchstaben "M" beispielhaft gezeigt ist (vgl. Figuren 4C und 4D). Solche Größenmarkierungen können sich auch in den Seitenteilen 46 befinden.

Darüber hinaus können optionale Markierungen 50 an der Sohlenfläche 16, in Nähe der länglichen Ausnehmungen 42 vorgesehen sein, wobei jeweils gleiche Markierungen 50 an den Seitenteilen 46 vorhanden sind. Diese zusätzlichen Markierungen 50 können die richtige Zuordnung des rechten und linken Seitenteils 46 zur jeweils richtigen Seite der Sohlenfläche 16 definieren.

Wie es die Fig. 4C deutlich erkennen lässt, können die unterschiedlich langen Seitenteile 46 bzw. Randteile 20 an beiden Längsseiten der Sohlenfläche 16 eingesteckt werden, und zwar zu beiden Seiten der Sohlenfläche 16. Die Abstände der Haken 44 an den unterschiedlich langen Seitenteilen 46 sind gleich, ebenso die Abstände der Ausnehmungen 42 voneinander. Zudem stimmen die Abstände der Haken 44 mit denen der Ausnehmungen 42 überein, so dass ein universelles Stecksystem 45 geschaffen ist, das eine universell für den linken und rechten Fuß verwendbare Anziehhilfe 10 bietet.

Da die Handhabungsschritte der in den Figuren 4A bis 4D gezeigten Anziehhilfe 10 mit dem Stecksystem 45 für die beiden randseitig der Sohlenfläche 16 einsteckbaren Seitenteile 46 in einigen Punkten von denen der einteiligen Anziehhilfe 10 gemäß Figuren 1 bis 3 abweichen, soll die Verwendung der Anziehhilfe 10 anhand der insgesamt zehn schematischen Ansichten der Figuren 5A bis 5J in aufeinander folgende Prozessschritten verdeutlicht werden; diese Prozessschritte zeigen die Verwendung der in den Figuren 4C und 4D gezeigten Variante der Anziehhilfe 10 im Zusammenhang mit dem Überziehen eines Kompressionsstrumpfes 28 über die Anziehhilfe 10.

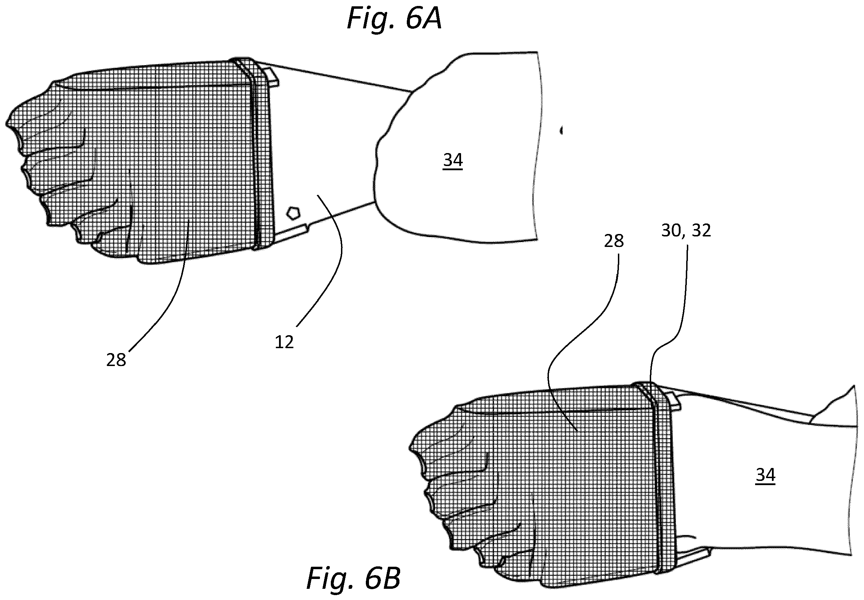

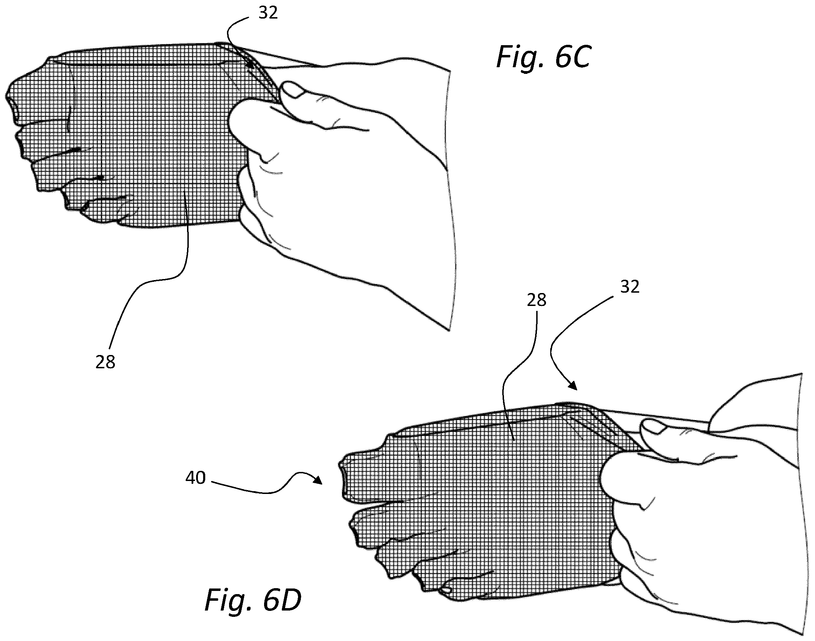

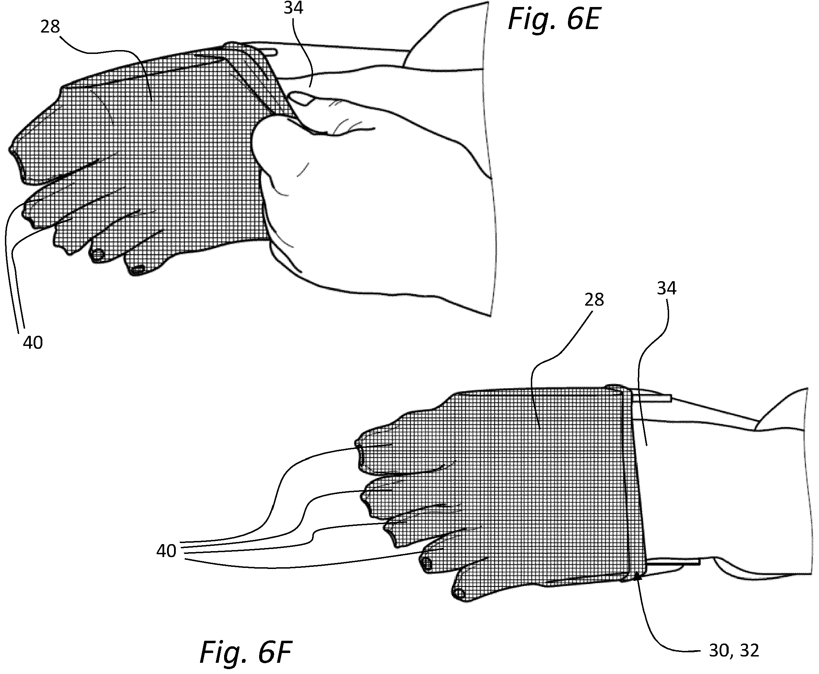

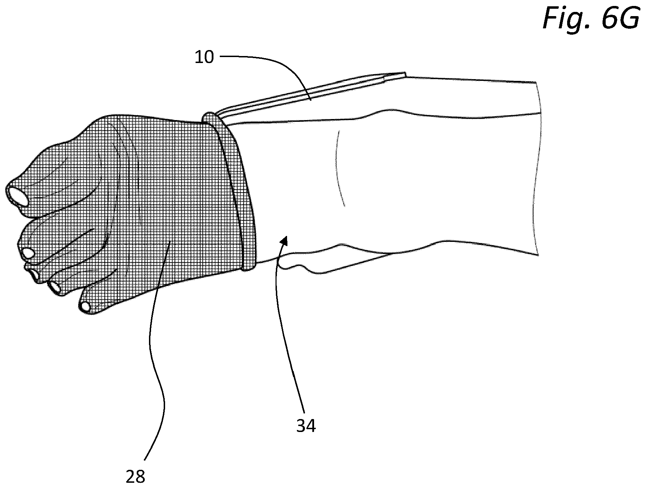

Die Fig. 6 zeigt zudem in insgesamt sieben schematischen Ansichten (Fig. 6A bis Fig. 6G) weitere aufeinander folgende Prozessschritte bei der Verwendung der in den Figuren 4C und 4D gezeigten Variante der Anziehhilfe 10 zum Überziehen des zuvor auf die Anziehhilfe 10 aufgezogenen Kompressionsstrumpfes 28 über einen menschlichen Fuß 34.

Die Fig. 5A verdeutlicht eine erste Phase der Verwendung der Anziehhilfe 10, bei der die Schablone 12 vorbereitet ist, indem dort noch keine Seitenteile 46 eingesteckt sind. Es wird zunächst nur die flache Schablone 12 selbst verwendet, und zwar als Anziehhilfe 10 für den linken Fuß. Die entsprechend passende Seite der Sohlenfläche 16 weist somit nach oben. Der Kompressions-Zehenstrumpf 28 für den linken Fuß kann somit über die Zehenabschnitte (hier bereits verdeckt) gezogen werden, bis der mittlere Teil der Schablone 12 bis über die vom Strumpf 28 bedeckten Ausnehmungen (vgl. Figuren 4C und 4D) gezogen ist.

Die Zehenschläuche 40 des Kompressions-Zehenstrumpfes 28 sind nun von den Zehenabschnitten 14 und dem Großzehabschnitt 18 der Schablone 12 ausgefüllt, wie dies die Fig. 5B verdeutlicht.

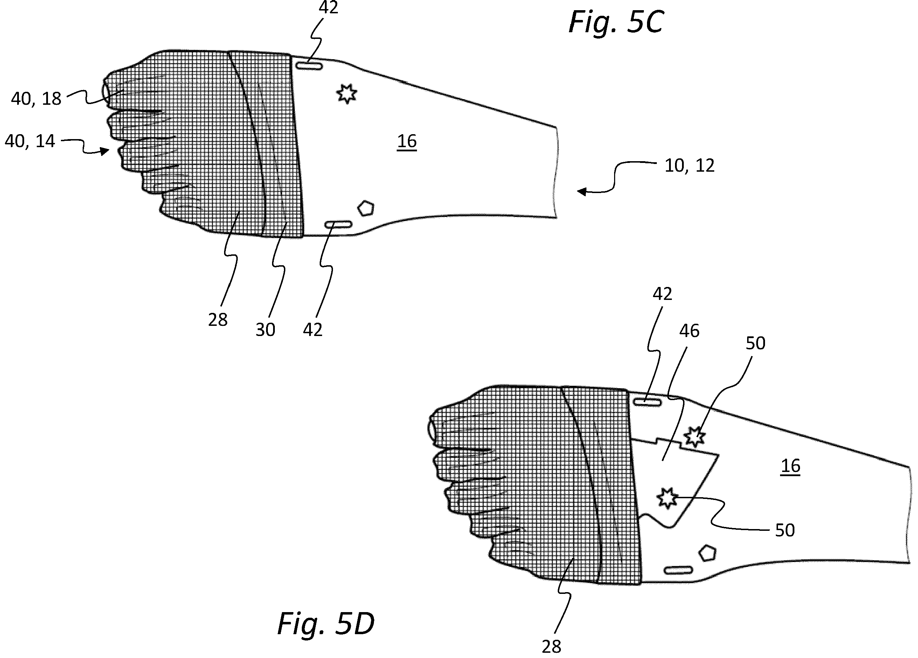

Das hintere Ende des Strumpfes 28, d.h. seine Einschlüpföffnung 30, wird nun wieder nach vorne umgeschlagen, und zwar in Richtung der Zehenabschnitte 14 und 18, damit zumindest die ersten links- und rechtsseitig an den Rändern der Sohlenfläche 16 befindlichen länglichen Ausnehmungen 42 freigelegt werden (vgl. Fig. 5C)

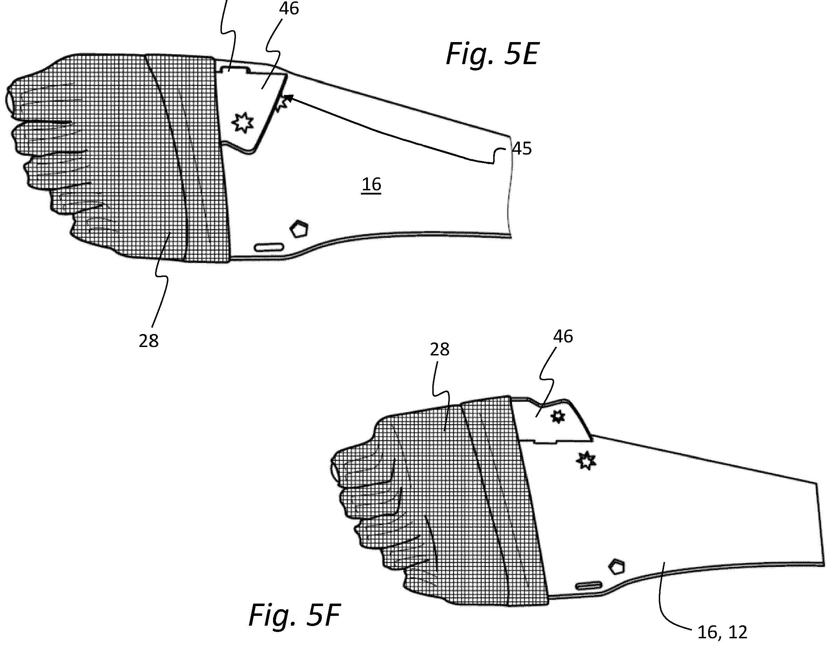

Anschließend wird das rechtsseitige Seitenteil 46 flach liegend unter den Strumpf 28 und seine Einschlüpföffnung 30 eingeschoben (vgl. Fig. 5D), wobei seine Haken 44 in Richtung nach außen und zu den Ausnehmungen 42 weisen. Die jeweiligen Symbole 50 des Seitenteils 46 und der Sohlenfläche 16, die in Nähe der Ausnehmung 42 an der rechten Randseite der Sohlenfläche 16 angeordnet sind, stimmen hierbei überein. Bei den Symbolen 50 handelt sich im gezeigten Ausführungsbeispiel um kleine sternförmige Durchbrüche im Seitenteil 46 sowie in der Sohlenfläche 16, wie dies in den Figuren 5D und 5E deutlich zu erkennen ist.

Gemäß Fig. 5E sollte anschließend das Seitenteil 46 in Richtung zur Außenseite der Sohlenfläche 16 geschoben werden, so dass das Stecksystem 45 in gewünschter Weise verwendet werden kann, indem die Haken 44 in die Ausnehmungen 42 gesteckt und das Seitenteil 46 gleichzeitig aufgerichtet wird, so dass es senkrecht auf der Sohlenfläche 16 steht und unter Einsatz des Stecksystems 45 mit der Schablone 12 im Eingriff steht (vgl. Fig. 5F). Der Strumpf 28 wird hierdurch durch das senkrecht stehende Seitenteil 46 etwas vorgespannt und von der Sohlenfläche 16 der Schablone 12 abgehoben.

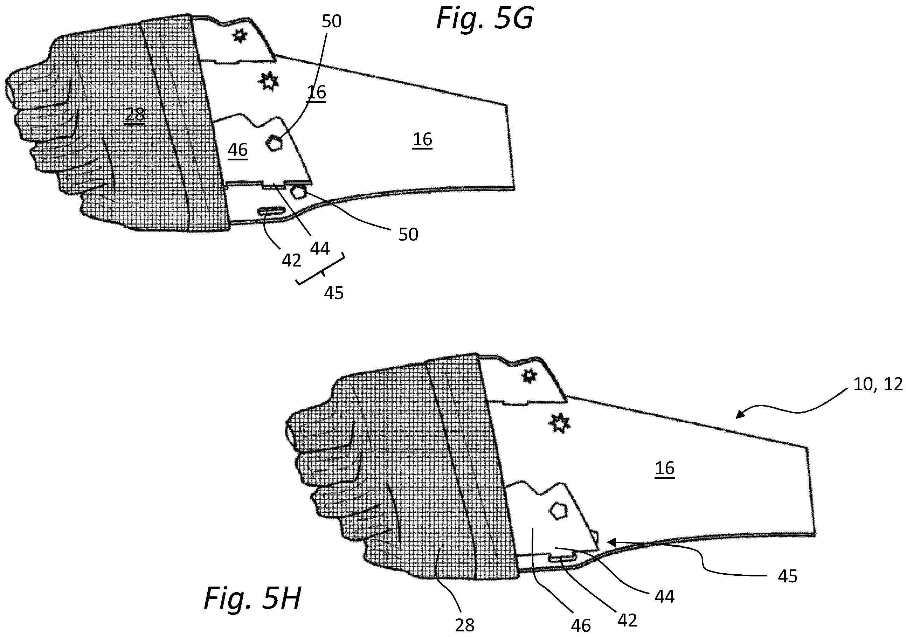

Nun kann das zweite, etwas kleinere Seitenteil 46 unter den Kompressionsstrumpf 28 geschoben werden (Fig. 5G), wobei wiederum die Haken 44 des Seitenteils 46 an seiner Unterseite zu den länglichen Ausnehmungen 42 am linken Außenrand der Sohlenfläche 16 weisen, damit das Stecksystem 45 bestimmungsgemäß verwendet werden kann. Die jeweiligen Symbole 50 des kleineren Seitenteils 46 und der Sohlenfläche 16, die in Nähe der Ausnehmung 42 an der linken Randseite der Sohlenfläche 16 angeordnet sind, stimmen hierbei überein. Bei den Symbolen 50 handelt sich im gezeigten Ausführungsbeispiel jeweils um kleine fünfeckige Durchbrüche im Seitenteil 46 sowie in der Sohlenfläche 16, wie dies in den Figuren 5G und 5H deutlich zu erkennen ist.

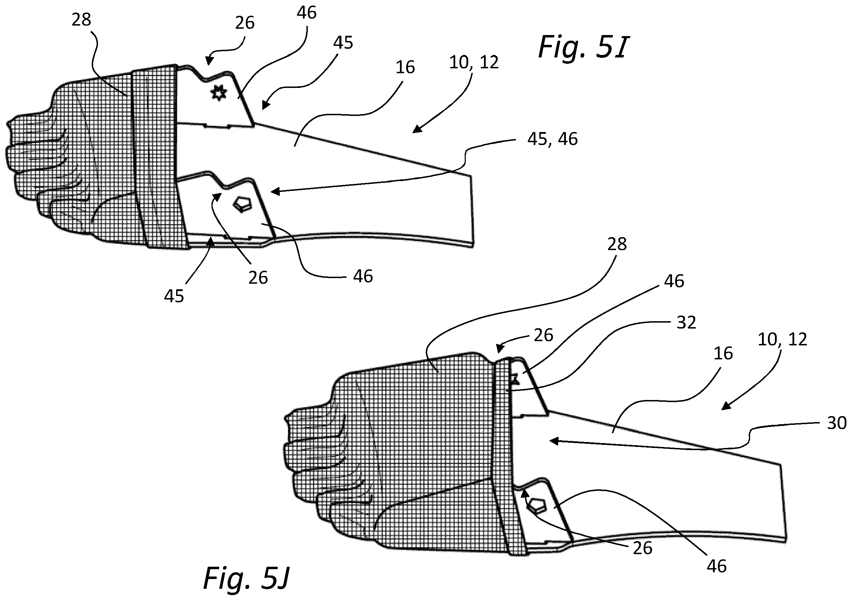

Gemäß Fig. 5H sollte anschließend das linke Seitenteil 46 in Richtung zur Innenseite der Sohlenfläche 16 geschoben werden, so dass das Stecksystem 45 in gewünschter Weise verwendet werden kann, indem die Haken 44 in die Ausnehmungen 42 gesteckt und das linke Seitenteil 46 gleichzeitig aufgerichtet wird, so dass es senkrecht auf der Sohlenfläche 16 steht und unter Einsatz des Stecksystems 45 mit der Schablone 12 im Eingriff steht (vgl. Fig. 5I). Der Strumpf 28 wird hierdurch durch beide senkrecht stehenden Seitenteile 46 stärker vorgespannt als zuvor (Fig. 5F) und von der Sohlenfläche 16 der Schablone 12 abgehoben.

Der Strumpf 28 kann nun mit seiner Einschlüpföffnung 30 weiter in Richtung zur Ferse gezogen werden, so dass sein aufgespannter Rand 32 in die Abstufungen 26 der Seitenteile 46 bzw. der beiden hochgezogenen Ränder 20 einhaken kann (Fig. 5J). Die Einschlüpföffnung 30 bleibt hierbei geöffnet und kann den Fuß aufnehmen, wenn dieser in die Öffnung 30 des Strumpfes 28 geschoben wird (vgl. Fig. 6A ff.).

Damit der linke Fuß 34 in den Strumpf 28 geschoben werden kann, wird die Schablone 12 sinnvollerweise mit dem rechten Fuß am Boden fixiert, indem dieser auf den Fersenabschnitt der Schablone 12 gestellt wird (vgl. Fig. 6A).

Der linke Fuß 34 kann somit durch den an den Abstufungen 26 der Seitenteile 46 zwischen den Seitenteilen 46 und der Sohlenfläche 16 aufgespannten Rand 32 der Einschlüpföffnung 30 in den Strumpf 28 hineingeschoben werden (Fig. 6B), wobei mit weiterem Hineinschieben der Rand 32 des Strumpfes 28 mit einer Hand festgehalten werden kann (vgl. Fig. 6C).

Die Zehen des Fußes 34 werden in die Zehenschläuche 40 einsortiert (Fig. 6D), so dass der Fuß 34 weiter in den Strumpf 28 hineingeschoben werden kann (Fig. 6E), wobei gleichzeitig der Strumpf 28 an seinem Rand 32 festgehalten werden sollte, um das Hineinschlüpfen zu unterstützen.

Wenn die Zehen des Fußes 34 ganz in die Zehenschläuche 40 des Strumpfes 28 hineingeschoben sind, kann der Rand 32 des Strumpfes 28 von den Abstufungen 26 abgehoben oder abgezogen werden (Fig. 6F), während gleichzeitig der rechte Fuß die Schablone 12 auf dem Boden fixiert. Der linke Fuß 34 kann nun mitsamt dem Strumpf 28 von der Anziehhilfe 10 gelöst werden (Fig. 6G), wonach schließlich noch das richtige und vorzugsweise faltenfreie Anziehen der Kompressionssocke 28 und das richtige Positionieren der Kompressionssocke 28 am Fuß 34 erfolgen kann.

Folgendes sei als ergänzender Hinweis zu den vorstehenden Ausführungen gegeben. Wenn auch im Zusammenhang mit den in den Figuren gezeigten Ausführungsvarianten und deren vorstehenden Beschreibungen oftmals oder auch generell von "schematischen" Darstellungen und Ansichten die Rede ist, so ist damit keineswegs gemeint, dass die Figurendarstellungen und deren Beschreibung hinsichtlich der Offenbarung der Erfindung von untergeordneter Bedeutung sein sollen. Der Fachmann ist durchaus in der Lage, aus den schematisch und abstrakt gezeichneten Darstellungen genug an Informationen zu entnehmen, die ihm das Verständnis der Erfindung erleichtern, ohne dass er etwa aus den gezeichneten und möglicherweise nicht exakt maßstabsgerechten Größenverhältnissen von Teilen der Vorrichtungen, deren Einzelheiten oder anderer gezeichneter Elemente in irgendeiner Weise in seinem Verständnis beeinträchtigt wäre. Die Figuren ermöglichen es dem Fachmann als Leser vielmehr, anhand der konkreter erläuterten Umsetzungen des erfindungsgemäßen Verfahrens und des konkreter erläuterten Aufbaus der erfindungsgemäßen Vorrichtung ein besseres Verständnis für den in den Ansprüchen sowie im allgemeinen Teil der Beschreibung zumindest hinsichtlich einiger Aspekte allgemeiner und/oder abstrakter formulierten Erfindungsgedanken abzuleiten.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 10: Anziehhilfe
- 12: Schablone
- 14: Zehenabschnitt
- 16: Sohlenfläche
- 18: Großzehabschnitt
- 20: Rand, hochgezogener Rand
- 22: Verankerungselement
- 24: Blechabschnitt, verlängerter Blechabschnitt
- 26: Abstufung, stufenartige Aussparung
- 28: Strumpf, Zehenstrumpf, Kompressionsstrumpf, Kompressions-Zehenstrumpf, Kompressionssocke
- 30: Strumpföffnung, Einschlüpföffnung
- 32: aufgespannter Rand (der Einschlüpföffnung)
- 34: Fuß, menschlicher Fuß
- 36: Bewegungsrichtung, Einschubrichtung
- 40: Zehenschlauch
- 42: Ausnehmung, längliche Ausnehmung
- 44: Haken
- 45: Stecksystem
- 46: Randabschnitt, Seitenteil
- 48: Markierung (L/R)
- 50: Symbol für Innenseite / Außenseite

## Patentansprüche

1. Anziehhilfe (10) zur Ermöglichung oder Erleichterung des Überstreifens eines Strumpfes oder Zehenstrumpfes (28) über einen menschlichen Fuß (34), insbesondere eines Kompressionsstrumpfes (28), der im entspannten Zustand ohne darin aufgenommenen Fuß (34) zumindest abschnittsweise ein kleineres Volumen bereithält oder einnimmt als im gedehnten, über den Fuß (34) gezogenen Zustand, welche Anziehhilfe (10) zumindest umfasst:
- eine Schablone (12) mit Umrisskonturen eines ausgestreckten und/oder flach auf einer Unterlage liegenden oder stehenden menschlichen Fußes (34) mit zumindest leicht voneinander abgespreizten und/oder seitlich voneinander beabstandeten Zehenabschnitten (14),
- zumindest an einer Längsseite der Sohlenfläche (16) der Schablone (12) einen zumindest abschnittsweise hochgezogenen Rand (20) sowie
- ein Halte-, Fixierungs- und/oder Verankerungselement (22) zur zumindest temporären Fixierung oder Abstützung der Schablone (12) beim Einführen des mit dem Strumpf (28) zu bedeckenden Fußes (34) gegen elastische Rückstellkräfte des über die Schablone (12) gezogenen und dabei entsprechend den Umrisskonturen der Schablone (12) gedehnten Strumpfes oder Kompressionsstrumpfes (28).

2. Anziehhilfe nach Anspruch 1, bei welcher der zumindest an einer Längsseite der Sohlenfläche (16) der Schablone (12) hochgezogene Rand (20) in Richtung eines Fersenbereiches höher gezogen ist als in einer Richtung zu den Zehenabschnitten (14).

3. Anziehhilfe nach Anspruch 1 oder 2, bei welcher der zumindest an einer Längsseite der Sohlenfläche (16) der Schablone (12) hochgezogene Rand (20) eine keil- oder dreieckförmige Kontur aufweist, deren kurze Seite im Bereich des Fersenbereiches rechtwinkelig zur Sohlenfläche (16) steht oder mit einer zur Sohlenfläche (16) senkrechten Achse einen spitzen Winkel einschließt.

4. Anziehhilfe nach einem der Ansprüche 1 bis 3, bei welcher der zumindest an einer Längsseite der Sohlenfläche (16) der Schablone (12) hochgezogene Rand (20) eine keil- oder dreieckförmige Kontur aufweist, deren lange Seite an die im Bereich des Fersenbereiches befindliche kurze Seite anschließt und in spitzem Winkel in Richtung zu einem randseitigen Zehenabschnitt (14) hin ausläuft, und/oder wobei der zum randseitig befindlichen Zehenabschnitt (14) hin auslaufende hochgezogene Rand (20) in den Zehenabschnitt (14) hineinreicht oder dort ausläuft.

5. Anziehhilfe nach einem der Ansprüche 1 bis 4, die an beiden gegenüberliegenden und voneinander beabstandeten Längsseiten der Sohlenfläche (16) der Schablone (12) jeweils zumindest abschnittsweise hochgezogene Ränder (20) aufweist.

6. Anziehhilfe nach einem der Ansprüche 1 bis 5, bei welcher der wenigstens eine hochgezogene Rand (20) oder zumindest einer der beiden hochgezogenen Ränder (20) an der oberen Kante der im Bereich des Fersenbereiches befindlichen kurzen Seite abgestuft ist, wodurch der Eckbereich im Übergang zur langen keilförmigen Seite des Randes (20) eine stufenartige Aussparung (26) bildet.

7. Anziehhilfe nach einem der Ansprüche 1 bis 6, bei welcher der wenigstens eine hochgezogene Rand (20) durch ein von der Schablone (12) abnehmbares Seitenteil (46) gebildet ist, das an definierter Position in die Sohlenfläche (16) der Schablone (12) einfügbar oder von dort entnehmbar ist.

8. Anziehhilfe nach Anspruch 7, bei der das wenigstens eine den hochgezogenen Rand (20) bildende Seitenteil (46) mittels eines Stecksystems (45) mit der Sohlenfläche (16) der Schablone (12) verbindbar ist, wobei das Stecksystem (45) insbesondere korrespondierende Ausnehmungen (42) in der Schablone (12) und damit korrespondierende Haken (44) an den Seitenteilen (46) umfasst.

9. Anziehhilfe nach Anspruch 8, bei der die Schablone (12) zu beiden Längsseiten jeweils gleichartige Stecksysteme (45) und/oder gleich dimensionierte Ausnehmungen (42) zur Aufnahme der Haken (44) der jeweils dort einfügbaren Seitenteile (46) aufweist.

10. Anziehhilfe nach Anspruch 9, bei der die Schablone (12) beidseitig für den linken oder den rechten Fuß (34) verwendbar ist.

11. Anziehhilfe nach einem der Ansprüche 1 bis 10, bei welcher das Halte-, Fixierungs- und/oder Verankerungselement (22) mit einem Gegenlager zusammenwirken kann und dort festlegbar ist.

12. Anziehhilfe nach einem der Ansprüche 1 bis 11, bei welcher ein von einem Fersenbereich ausgehender Schaftabschnitt verlängert ist, wodurch die Anziehhilfe (10) auch für Wadenstrümpfe, insbesondere für Kompressions-Wadenstrümpfe geeignet ist.

13. Verfahren zum erleichterten Anziehen eines Strumpfes, eines Zehenstrumpfes oder eines Kompressionsstrumpfes (28) und/oder zur Unterstützung beim Überstreifen eines Kompressionsstrumpfes (28) oder Kompressions-Zehenstrumpfes über einen menschlichen Fuß (34) unter Verwendung einer mechanischen Anziehhilfe (10), insbesondere unter Verwendung einer Anziehhilfe (10) gemäß einem der Ansprüche 1 bis 12, wobei das Verfahren zumindest die folgenden Verfahrensschritte vorsieht:
- Überstreifen des Kompressionsstrumpfes (28) oder Kompressions-Zehenstrumpfes (28) über eine Schablone (12), welche Umrisskonturen eines ausgestreckten und/oder flach auf einer Unterlage liegenden oder stehenden menschlichen Fußes (34) mit zumindest leicht voneinander abgespreizten und/oder seitlich voneinander beabstandeten Zehenabschnitten (14) aufweist, wobei die Zehenabschnitte (14) der Schablone (12) in die Zehenschläuche (40) des Kompressionsstrumpfes (28) oder Kompressions-Zehenstrumpfes (28) eintauchen,
- Positionieren und Arretieren und/oder Fixieren der in den Kompressionsstrumpf (28) oder Kompressions-Zehenstrumpf (28) eingetauchten Schablone (12) an einem Gegenlager, das ein Ausweichen der Schablone (12) zumindest in einer vom Fersenbereich zu den Zehenspitzen weisenden Einschubrichtung (36) verhindert,
- Einschieben des mit dem Kompressionsstrumpfes (28) oder Kompressions-Zehenstrumpf (28) auszustattenden menschlichen Fußes (34) in den durch die Schablone (12) in Form gebrachten und durch wenigstens einen an einer Seite hochgezogenen Rand (20) oder durch die randseitig hochgezogenen Ränder (20) der Schablone (12) in einer Richtung entgegen der Einschubrichtung (36) geöffneten Kompressionsstrumpf (28) oder Kompressions-Zehenstrumpf (28) oder in den vorderen, den Fuß (34) bedeckenden Bereich des Kompressionsstrumpfes (28), wobei der Kompressionsstrumpf (28) oder Kompressions-Zehenstrumpf (28) durch die keilförmige Gestaltung des Randes (20) oder der randseitig hochgezogenen Ränder (20) der Schablone (12) dort gehalten wird,
- Abziehen des vollständig oder weitgehend durch die Zehen und den Fuß (34) ausgefüllten Kompressionsstrumpfes (28) oder Kompressions-Zehenstrumpfes (28) von der Schablone (12) durch oder unter gleichzeitigem Lösen der am Rand (20) oder an den hochgezogenen Rändern (20) der Schablone (12) gehaltenen Bereiche des Kompressionsstrumpfes (28) oder Kompressions-Zehenstrumpfes (28).

14. Verfahren zum erleichterten Anziehen eines Strumpfes, eines Zehenstrumpfes oder eines Kompressionsstrumpfes (28) und/oder zur Unterstützung beim Überstreifen eines Kompressionsstrumpfes (28) oder Kompressions-Zehenstrumpfes über einen menschlichen Fuß (34) unter Verwendung einer mechanischen Anziehhilfe (10), insbesondere unter Verwendung einer Anziehhilfe (10) gemäß einem der Ansprüche 7 bis 12, wobei das Verfahren zumindest die folgenden Verfahrensschritte vorsieht:
- Überstreifen des Kompressionsstrumpfes (28) oder Kompressions-Zehenstrumpfes (28) über eine Schablone (12), welche Umrisskonturen eines ausgestreckten und/oder flach auf einer Unterlage liegenden oder stehenden menschlichen Fußes (34) mit zumindest leicht voneinander abgespreizten und/oder seitlich voneinander beabstandeten Zehenabschnitten (14) aufweist, wobei die Zehenabschnitte (14) der Schablone (12) in die Zehenschläuche (40) des Kompressionsstrumpfes (28) oder Kompressions-Zehenstrumpfes (28) eintauchen,
- Positionieren und Arretieren und/oder Fixieren der in den Kompressionsstrumpf (28) oder Kompressions-Zehenstrumpf (28) eingetauchten Schablone (12) an einem Gegenlager und/oder auf dem Boden, so das ein Ausweichen der Schablone (12) zumindest in einer vom Fersenbereich zu den Zehenspitzen weisenden Einschubrichtung (36) verhindert wird,
- Einfügen eines von zwei Seitenteilen (46) in den Strumpf (28) und Aufrichten des Seitenteils (46) durch Einstecken der an den Seitenteilen (46) befindlichen Haken (44) in korrespondierende längliche Ausnehmungen (42), die sich an einer Längsseite der Schablone (12) befinden,
- Einfügen des zweiten Seitenteils (46) in den Strumpf (28) und Aufrichten des zweiten Seitenteils (46) durch Einstecken der am Seitenteil (46) befindlichen Haken (44) in korrespondierende längliche Ausnehmungen (42), die sich an der zweiten Längsseite der Schablone (12) befinden,
- Einschieben des mit dem Kompressionsstrumpfes (28) oder Kompressions-Zehenstrumpf (28) auszustattenden menschlichen Fußes (34) in den durch die Schablone (12) in Form gebrachten und durch die randseitig hochgezogenen Ränder (20) der in die Schablone (12) eingesteckten Seitenteile (46) in einer Richtung entgegen der Einschubrichtung (36) geöffneten Kompressionsstrumpf (28) oder Kompressions-Zehenstrumpf (28) oder in den vorderen, den Fuß (34) bedeckenden Bereich des Kompressionsstrumpfes (28), wobei der Kompressionsstrumpf (28) oder Kompressions-Zehenstrumpf (28) durch die keilförmige Gestaltung der randseitig hochgezogenen Ränder (20) der in die Schablone (12) eingesteckten Seitenteile (46) dort gehalten wird,
- Abziehen des vollständig oder weitgehend durch die Zehen und den Fuß (34) ausgefüllten Kompressionsstrumpfes (28) oder Kompressions-Zehenstrumpfes (28) von der Schablone (12) durch oder unter gleichzeitigem Lösen der an den hochgezogenen Rändern (20) der in die Schablone (12) eingesteckten Seitenteile (46) gehaltenen Bereiche des Kompressionsstrumpfes (28) oder Kompressions-Zehenstrumpfes (28).

15. Verfahren nach Anspruch 14, bei dem die Seitenteile (46) unter Nutzung der jeweiligen Stecksysteme (45), bestehend aus Haken (44) und diese aufnehmende Ausnehmungen (42) in die Schablone (12) eingesteckt werden, wenn der Kompressionsstrumpf (28) oder Kompressions-Zehenstrumpf (28) bereits über diese gespannt ist, wobei ein Rand (32) der Strumpföffnung (30) in stufenartige Aussparungen (26) eingehängt wird, die sich an den von der Sohlenfläche (16) der Schablone (12) wegweisenden Rückseiten der Seitenteile (46) befinden.

## Claims

1. A donning aid (10) used to enable or facilitate slipping a stocking or toe stocking (28) onto a human foot (34), in particular, a compression stocking (28), which, in a relaxed state without foot (34) received therein, at least in some sections offers or takes up a smaller volume than in the stretched state when pulled over the foot (34), which donning aid (10) comprises at least:
- a shell (12) with contours of a human foot (34) that is stretched and/or lying or standing flat on a support with toe sections (14) at least slightly spread apart and/or laterally spaced apart from each other,
- an edge (20) at least at one longitudinal side of the sole surface (16) of the shell (12), which edge (20) is at least in some sections raised, as well as
- a holding, securing and/or fastening element (22) to secure or support the shell (12), at least temporarily while the foot (34) to be covered with the stocking (28) is being inserted, against elastic restoring forces of the stocking or compression stocking (28) that is pulled over the shell (12) and thereby stretched corresponding to the contours of the shell (12).

2. The donning aid according to claim 1, in which the edge (20) that is raised at least at one longitudinal side of the sole surface (16) of the shell (12) is raised higher toward a heel area than in a direction toward the toe sections (14).

3. The donning aid according to claim 1 or 2, in which the edge (20) that is raised at least at one longitudinal side of the sole surface (16) of the shell (12) has a wedge-shaped or triangular contour, the short side of which is at right angles to the sole surface (16) in the area of the heel area, or which encloses an acute angle with an axis that is perpendicular to the sole surface (16).

4. The donning aid according to one of the claims 1 to 3, in which the edge (20) that is raised at least at one longitudinal side of the sole surface (16) of the shell (12) has a wedge-shaped or triangular contour, the long side of which connects to the short side that is situated in the area of the heel area, and which tapers off in an acute angle toward a toe section (14) at the edge side, and/or wherein the raised edge (20) tapering off toward the toe section (14) situated at the edge side extends into the toe section (14) or tapers off there.

5. The donning aid according to one of the claims 1 to 4, which has edges (20) that are raised at least in some sections at each of both oppositely situated and spaced-apart longitudinal sides of the sole surface (16) of the shell (12).

6. The donning aid according to one of the claims 1 to 5, in which the at least one raised edge (20) or at least one of the two raised edges (20) is stepped at the upper rim of the short side situated in the area of the heel area, whereby the corner area forms a steplike recess (26) in the transition to the long, wedge-shaped side of the edge (20).

7. The donning aid according to one of the claims 1 to 6, in which the at least one raised edge (20) is formed by a detachable side part (46) of the shell (12), which side part (46) is introducible into or removable from the sole surface (16) of the shell (12) at a defined position.

8. The donning aid according to claim 7, in which the at least one side part (46) forming the raised edge (20) is connectable to the sole surface (16) of the shell (12) by an interlocking assembly system (45), wherein the interlocking assembly system (45) comprises, in particular, corresponding recesses (42) in the shell (12) and therewith corresponding latches (44) at the side parts (46).

9. The donning aid according to claim 8, in which the shell (12) has interlocking assembly systems (45) of the same type and/or equally dimensioned recesses (42) at each of the two longitudinal sides to receive the latches (44) of the in each case therein insertable side parts (46).

10. The donning aid according to claim 9, in which the shell (12) is usable on both sides for the left or the right foot (34).

11. The donning aid according to one of the claims 1 to 10, in which the holding, securing and/or fastening element (22) can interact with a counter support and is securable there.

12. The donning aid according to one of the claims 1 to 11, in which a leg section extending from a heel area is made longer, whereby the donning aid (10) is also suitable for calf stockings, in particular, for calf compression stockings.

13. A method for the easier donning of a stocking, a toe stocking, or a compression stocking (28) and/or for the support when slipping a compression stocking (28) or toe compression stocking onto a human foot (34) using a mechanical donning aid (10), in particular, using a donning aid (10) according to one of the claims 1 to 12, wherein the method provides at least the following method steps:
- slipping the compression stocking (28) or toe compression stocking (28) onto a shell (12) having the contours of a human foot (34) that is stretched and/or lying or standing flat on a support with toe sections (14) at least slightly spread apart and/or laterally spaced apart from each other, wherein the toe sections (14) of the shell (12) slip into the toe sleeves (40) of the compression stocking (28) or toe compression stocking (28),
- positioning and locating and/or securing the shell (12) to a counter support when the shell has been slipped into the compression stocking (28) or toe compression stocking (28), which counter support prevents the shell (12) from yielding at least in an insertion direction (36) facing from the heel area to the toe tips,
- inserting the human foot (34) to be fitted with the compression stocking (28) or toe compression stocking (28) into the compression stocking (28) or toe compression stocking (28), with the compression stocking (28) or toe compression stocking (28) being molded by the shell (12) and being opened in a direction against the insertion direction (36) by an edge (20) of the shell (12) that is raised at at least one side or by the edges (20) of the shell (12) that are raised at the edge sides, or into the front area of the compression stocking (28) covering the foot (34), wherein the compression stocking (28) or toe compression stocking (28) is held there by the wedge-shaped design of the edge (20) or of the edges (20) of the shell (12) that are raised at the edge sides,
- slipping the compression stocking (28) or toe compression stocking (28), which is completely or largely filled out by the toes and the foot (34), off of the shell (12) by or with simultaneously removing the areas of the compression stocking (28) or toe compression stocking (28) that are being held at the edge (20) or at the raised edges (20) of the shell (12).

14. A method used for the easier donning of a stocking, a toe stocking, or a compression stocking (28) and/or for the support when slipping a compression stocking (28) or a toe compression stocking onto a human foot (34) using a mechanical donning aid (10), in particular, using a donning aid (10) according to one of the claims 7 to 12, wherein the method provides at least the following method steps:
- slipping the compression stocking (28) or toe compression stocking (28) onto a shell (12) having the contours of a human foot (34) that is stretched and/or lying or standing flat on a support with toe sections (14) at least slightly spread apart and/or laterally spaced apart from each other, wherein the toe sections (14) of the shell (12) slip into the toe sleeves (40) of the compression stocking (28) or toe compression stocking (28),
- positioning and locating and/or securing the shell (12) to a counter support and/or on the floor when the shell has been slipped into the compression stocking (28) or toe compression stocking (28) such that the shell (12) is prevented from yielding at least in an insertion direction (36) facing from the heel area to the toe tips,
- introducing one of two side parts (46) into the stocking (28) and erecting the side part (46) by slotting the latches (44) situated on the side parts (46) into corresponding oblong recesses (42) situated at a longitudinal side of the shell (12),
- introducing the second side part (46) into the stocking (28) and erecting the second side part (46) by slotting the latches (44) situated on the side parts (46) into corresponding oblong recesses (42) situated at the second longitudinal side of the shell (12),
- inserting the human foot (34) to be fitted with the compression stocking (28) or toe compression stocking (28) into the compression stocking (28) or toe compression stocking (28), with the compression stocking (28) or toe compression stocking (28) being molded by the shell (12) and being opened in a direction against the insertion direction (36) by the edges (20) of the side parts (46) that are slotted into the shell (12) with the edges (20) that are raised at the edge sides, or into the front area of the compression stocking (28) covering the foot (34), wherein the compression stocking (28) or toe compression stocking (28) is held there by the wedge-shaped design of the edges (20) of the side parts (46) that are slotted into the shell (12) with the edges (20) raised at the edge sides,
- slipping the compression stocking (28) or toe compression stocking (28), which is completely or largely filled out by the toes and the foot (34), off of the shell (12) by or with simultaneously removing the areas of the compression stocking (28) or toe compression stocking (28) that are being held at the raised edges (20) of the side parts (46) that are slotted into the shell (12).

15. The method according to claim 14, in which the side parts (46) are slotted into the shell (12) using the particular interlocking assembly systems (45), which consist of latches (44) and recesses (42) to receive the latches (44), when the compression stocking (28) or toe compression stocking (28) is already stretched over the shell (12), wherein a border (32) of the stocking opening (30) is fitted into steplike recesses (26) that are situated at the back sides of the side parts (46) facing away from the sole surface (16) of the shell (12).

## Revendications

1. Aide à l'habillage (10) pour permettre ou faciliter l'enfilage d'un bas ou d'un bas pour orteils (28) sur un pied humain (34), notamment d'un bas de contention (28), qui, à l'état détendu, vide de pied (34), fournit ou occupe au moins en partie un volume moins important qu'à l'état tendu et tiré sur le pied (34), laquelle aide à l'habillage (10) comprend au moins :
- un gabarit (12) doté de contours d'un pied humain (34) tendu et/ou posé à plat ou debout sur un support, doté de sections d'orteils (14) légèrement écartées les unes des autres et/ou séparées latéralement les unes des autres,
- un bord (20) relevé au moins partiellement au moins sur un côté longitudinal de la surface de semelle (16) du gabarit (12), ainsi qu'
- un élément de maintien, de fixation et/ou d'ancrage (22) pour fixer ou soutenir au moins temporairement le gabarit (12), au moment d'introduire le pied (34) à recouvrir avec le bas (28), contre les forces de rappel élastiques du bas ou bas de contention (28) tiré sur le gabarit (12) et tendu à cet égard en fonction des contours dudit gabarit (12).

2. Aide à l'habillage selon la revendication 1, dans laquelle le bord (20) relevé au moins sur un côté longitudinal de la surface de semelle (16) du gabarit (12) est, dans le sens d'une zone de talon, relevé plus haut que dans un sens orienté vers les sections d'orteils (14).

3. Aide à l'habillage selon la revendication 1 ou 2, dans laquelle le bord (20) relevé au moins sur un côté longitudinal de la surface de semelle (16) du gabarit (12) présente un contour cunéiforme ou triangulaire dont le côté court est, dans la zone du talon, perpendiculaire à la surface de semelle (16), ou constitue un angle aigu avec un axe vertical à la surface de semelle (16).

4. Aide à l'habillage selon l'une quelconque des revendications 1 à 3, dans laquelle le bord (20) relevé au moins sur un côté longitudinal de la surface de semelle (16) du gabarit (12) présente un contour cunéiforme ou triangulaire dont le côté long se raccorde au côté court se trouvant dans la zone du talon et se termine sous un angle aigu en direction d'une section d'orteil (14) située du côté du bord, et/ou le bord (20) relevé se terminant vers la section d'orteil (14) située du côté du bord pénètre dans la section d'orteil (14) ou s'y termine.

5. Aide à l'habillage selon l'une quelconque des revendications 1 à 4, qui présente, sur les deux côtés longitudinaux opposés et espacés l'un de l'autre de la surface de semelle (16) du gabarit (12), respectivement des bords (20) relevés au moins partiellement.

6. Aide à l'habillage selon l'une quelconque des revendications 1 à 5, dans laquelle ledit au moins un bord (20) relevé ou au moins l'un des deux bords (20) relevés est gradué sur le bord supérieur du côté court situé dans la zone du talon, la zone de coin formant de ce fait un évidement (26) en forme de gradin dans la transition vers le côté long cunéiforme du bord (20).

7. Aide à l'habillage selon l'une quelconque des revendications 1 à 6, dans laquelle ledit au moins un bord (20) relevé est formé par une partie latérale (46) détachable du gabarit (12), qui peut être insérée, à une position définie, dans la surface de semelle (16) du gabarit (12) ou en être retirée.

8. Aide à l'habillage selon la revendication 7, dans laquelle ladite au moins une partie latérale (46) formant le bord (20) relevé peut être reliée à la surface de semelle (16) du gabarit (12) au moyen d'un système d'enfichage (45), ledit système d'enfichage (45) comprenant notamment des évidements (42) correspondants dans le gabarit (12) et des crochets (44) y correspondant sur les parties latérales (46).

9. Aide à l'habillage selon la revendication 8, dans laquelle le gabarit (12) présente des systèmes d'enfichage (45) respectivement de même type des deux côtés longitudinaux et/ou des évidements (42) de même dimension destinés à recevoir les crochets (44) des parties latérales (46) pouvant respectivement y être insérées.

10. Aide à l'habillage selon la revendication 9, dans laquelle le gabarit (12) peut être utilisé des deux côtés pour le pied (34) gauche ou droit.

11. Aide à l'habillage selon l'une quelconque des revendications 1 à 10, dans laquelle l'élément de maintien, de fixation et/ou d'ancrage (22) peut coopérer avec un contre-appui et y être fixé.

12. Aide à l'habillage selon l'une quelconque des revendications 1 à 11, dans laquelle une section de tige partant d'une zone de talon est prolongée, ladite aide à l'habillage (10) étant ainsi également adaptée aux bas de mollet, notamment aux bas de contention de mollet.

13. Procédé destiné à faciliter l'enfilage d'un bas, d'un bas d'orteil ou d'un bas de contention (28) et/ou pour aider à passer un bas de contention (28) ou un bas de contention pour orteils sur un pied humain (34) en utilisant une aide mécanique à l'habillage (10), notamment en utilisant une aide à l'habillage (10) selon l'une quelconque des revendications 1 à 12, ledit procédé prévoyant au moins les étapes suivantes :
- enfiler le bas de contention (28) ou bas de contention pour orteils (28) sur un gabarit (12) qui présente les contours d'un pied humain (34) tendu et/ou posé à plat sur un support ou debout avec des sections d'orteils (14) au moins légèrement écartées les unes des autres et/ou espacées latéralement les unes des autres, les sections d'orteils (14) du gabarit (12) plongeant dans les tubes d'orteils (40) du bas de contention (28) ou bas de contention pour orteils (28),
- positionner et bloquer et/ou fixer le gabarit (12) immergé dans le bas de contention (28) ou bas de contention pour orteils (28) sur un contre-appui qui empêche le gabarit (12) de se dérober au moins dans un sens d'insertion (36) orienté de la zone du talon vers la pointe des pieds,
- insérer le pied humain (34) à pourvoir du bas de contention (28) ou bas de contention pour orteils (28), dans le bas de contention (28) ou bas de contention pour orteils (28) mis en forme par le gabarit (12) et ouvert par au moins un bord (20) relevé d'un côté ou par les bords (20), relevés du côté du bord, du gabarit (12) dans un sens opposé au sens d'insertion (36) ou dans la partie avant du bas de contention (28) qui recouvre le pied (34), ledit bas de contention (28) ou bas de contention pour orteils (28) y étant maintenu par la configuration cunéiforme du bord (20) ou des bords (20), relevés du côté du bord, du gabarit (12)
- retirer le bas de contention (28) ou bas de contention pour orteils (28), complètement ou largement rempli par les orteils et le pied (34), du gabarit (12) par ou en libérant simultanément les zones du bas de contention (28) ou bas de contention pour orteils (28) maintenues sur le bord (20) ou sur les bords (20) relevés du gabarit (12).

14. Procédé destiné à faciliter l'enfilage d'un bas, d'un bas pour orteils ou d'un bas de contention (28) et/ou pour aider à passer un bas de contention (28) ou bas de contention pour orteils sur un pied humain (34) en utilisant une aide mécanique à l'habillage (10), notamment en utilisant une aide à l'habillage (10) selon l'une quelconque des revendications 7 à 12, ledit procédé prévoyant au moins les étapes suivantes :
- enfiler le bas de contention (28) ou le bas de contention pour orteils (28) sur un gabarit (12) qui présente les contours d'un pied humain (34) tendu et/ou posé à plat sur un support ou debout, avec des sections d'orteil (14) au moins légèrement écartées les unes des autres et/ou espacées latéralement les unes des autres, lesdites sections d'orteils (14) du gabarit (12) plongeant dans les tubes d'orteils (40) du bas de contention (28) ou bas de contention pour orteils (28),
- positionner et bloquer et/ou fixer le gabarit (12) immergé dans le bas de contention (28) ou bas de contention pour orteils (28) sur un contre-appui et/ou sur le sol, de sorte à empêcher le gabarit (12) de se dérober au moins dans un sens d'insertion (36) orienté de la zone du talon vers la pointe des pieds,
- introduire l'une de deux parties latérales (46) dans le bas (28) et relever ladite partie latérale (46) en insérant le crochet (44) situé sur les parties latérales (46) dans des évidements (42) de forme allongée correspondants qui se trouvent sur un côté longitudinal du gabarit (12),
- introduire la deuxième partie latérale (46) dans le bas (28) et relever ladite deuxième partie latérale (46) en insérant le crochet (44) situé sur la partie latérale (46) dans des évidements (42) de forme allongée correspondants qui se trouvent sur le deuxième côté longitudinal du gabarit (12),
- insérer le pied humain (34) à pourvoir du bas de contention (28) ou bas de contention pour orteils (28), dans le bas de contention (28) ou bas de contention pour orteils (28) mis en forme par le gabarit (12) et ouvert par les bords (20), relevés du côté du bord, des parties latérales (46) insérées dans le gabarit (12) dans un sens opposé au sens d'insertion (36) ou dans la partie avant du bas de contention (28) qui recouvre le pied (34), ledit bas de contention (28) ou bas de contention pour orteils (28) y étant maintenu par la configuration cunéiforme des bords (20), relevés du côté du bord, des parties latérales (46) insérées dans le gabarit (12),
- retirer le bas de contention (28) ou bas de contention pour orteils (28), complètement ou largement rempli par les orteils et le pied (34), du gabarit (12) par ou en libérant simultanément les zones du bas de contention (28) ou bas de contention pour orteils (28) maintenues sur les bords (20) relevés des parties latérales (46) insérées dans le gabarit (12).

15. Procédé selon la revendication 14, dans lequel les parties latérales (46) sont insérées dans le gabarit (12) en utilisant les systèmes d'enfichage (45) constitués de crochets (44) et d'évidements (42) les recevant, lorsque le bas de contention (28) ou bas de contention pour orteils (28) est déjà tendu sur celui-ci, un bord (32) de l'ouverture du bas (30) étant accroché dans des évidements (26) en forme de gradins qui se trouvent sur les côtés verso des parties latérales (46) orientés à l'opposé de la surface de semelle (16) du gabarit (12).
